Europäisches Patentamt

European Patent Office

Office européen des brevets

(19)

(11) **EP 0 511 108 B1**

(12) **FASCICULE DE BREVET EUROPEEN**

(45) Date de publication et mention
de la délivrance du brevet:
**12.08.1998 Bulletin 1998/33**

(51) Int Cl.⁶: **C12N 1/18**

(21) Numéro de dépôt: **92401168.7**

(22) Date de dépôt: **23.04.1992**

(54) **Nouvelles souches de levure de panification et leur procédé d'obtention, nouvelles levures fraîche et sèche correspondantes**

Backhefestämme, deren Verfahren zur Herstellung, und zugehörige trockene und frische Hefe

Baker's yeast strains, their process of obtention, corresponding fresh and dry yeasts

(84) Etats contractants désignés:
**AT BE CH DE DK ES FR GB GR IT LI LU NL PT SE**

(30) Priorité: **23.04.1991 FR 9105008**

(43) Date de publication de la demande:
**28.10.1992 Bulletin 1992/44**

(83) **Déclaration conformément à la règle 28(4) CBE
(solution de l'expert)**

(73) Titulaire: **LESAFFRE et Cie
F-75001 Paris (FR)**

(72) Inventeurs:
  • **Loiez, Annie
    F-59000 Lille (FR)**
  • **Clément, Philippe
    F-59100 Roubaix (FR)**
  • **Colavizza, Didier
    F-59163 Condé Sur L'Escaut (FR)**

(74) Mandataire: **Koch, Gustave et al
Cabinet PLASSERAUD
84, rue d'Amsterdam
75440 Paris Cédex 09 (FR)**

(56) Documents cités:
**EP-A- 0 008 554          EP-A- 0 128 524
EP-A- 0 229 976          EP-A- 0 306 107
BE-A- 895 475            BE-A- 895 475
GB-A- 1 539 211          GB-A- 1 590 830**

• **WORLD PATENTS INDEX LATEST Week 9120,
Derwent Publications Ltd., London, GB; AN
91-144175 [20]**
• **CHEMICAL ABSTRACTS DATABASE
COLUMBUS OHIO US Abstract
No.95(21):183746B Carlson, Marian; Osmond,
Barbara C.; Botstein, D 'Mutants of yeast
defective in sucrose ...'**
• **BIOSIS PREVIEWS DATABSE,Philadelphia
ABSTRACT NO.90:48239 CARU M; CIFUENTES
B; PINCHEIRA G; JIMENEZ A 'MOLECULAR
CLONING AND EXPRESSION IN ...'**
• **Nagodawithana et al. (1990) Yeast Selection for
Baking, in Panchal (Ed.) Yeast Strain Selections.
NY: Marcel Dekker, pp.139-184.**
• **Trivedi et al. (1986) Bakers' Yeast. CRC Crit.
Review in Biotechnol. 4(1), pp.75-109.**
• **Rothstein (1983), One-Step Gene Disruption in
Yeast. Methods in Enzymology, 101, pp.202-211.**

**Description**

L'invention a pour objet de nouvelles souches de levure de panification à large spectre, c'est-à-dire performantes à la fois sur pâtes sans sucre et sur pâtes fortement sucrées ainsi qu'un procédé d'obtention de ces nouvelles souches; elle vise également, en tant que produits industriels nouveaux, les levures de panification fraîche ou sèche obtenues à partir de ces souches.

On connaît déjà des souches adaptées à la fermentation du maltose et relativement osmotolérantes, c'est-à-dire présentant un assez large spectre d'application.

A cet égard, on rappelle le brevet français N° 7739149, le brevet européen N° 79 400555.3 demandé le 03.08.1979 et accordé sous le N° O 008 554 et le brevet américain N° 4.396.632 du 02.08.1983, tous trois au nom de la Société Demanderesse.

Ces trois brevets correspondent à des aspects différents de l'exploitation d'un procédé reproductible d'obtention par hybridation de nouvelles souches à large spectre, ledit procédé reposant sur le choix judicieux des souches parentes et l'emploi de tests performants de sélection des nouvelles souches à large spectre ainsi construites.

Il a également été proposé de préparer des souches à large spectre en transformant une souche de départ par intégration de gènes qui codent pour des enzymes permettant la fermentation du maltose (demande de brevet européen N° 88 201870.8 du 01.09.1988 publié sous le N° 0 306 107). Les résultats industriels obtenus tels qu'ils apparaissent à la lecture de la description sont médiocres. Ainsi, le gain obtenu sur pâte normale selon le tableau 6, page 20, par intégration dans une souche osmotolérante d'un vecteur intégratif dépourvu de DNA hétérologue codant et contenant des gènes MAL est de 18%, ce qui est largement insuffisant pour pouvoir qualifier la souche obtenue comme étant à large spectre. En effet, l'écart d'activité sur pâte normale (pâte sans sucre) entre une souche osmotolérante très active sur pâte sucrée, et une souche très bien adaptée à la fermentation du maltose peut atteindre des rapports de l'ordre de 1 à 2. Or, il apparaît à la lecture du brevet que cette construction est la seule pouvant être considérée comme stable et susceptible de conduire à des levures de panification pouvant être commercialisées. Toujours à la même page 20, il est indiqué que les autres constructions décrites --qui ne semblent pas pouvoir conduire à des levures de panification commercialisables mais qui ont conduit à des gains théoriques de l'ordre de 30%, ce qui est encore insuffisant--, permettent de penser que des progrès du même ordre pourront être obtenus en augmentant le nombre de copies des gènes intégrés sans DNA hétérologue codant; c'est une hypothèse qui reste toutefois à vérifier.

Ceci étant, les souches de levures rapides, adaptées au maltose utilisées industriellement depuis une vingtaine d'années, sont des souches pour lesquelles, comme leur nom l'indique, la fermentation du maltose n'est guère réprimée par la présence de glucose et est peu dépendante de l'induction par le maltose. Ces souches ont été construites par hybridation. L'hybridation, quand elle résulte uniquement de croisements menés au hasard, est une méthode dont les résultats sont souvent considérés comme non prévisibles et non reproductibles. Cela n'est plus vrai dès lors que les croisements ne sont pas menés au hasard et que des tests de sélection efficace existent. L'expérience montre que l'obtention de souches très bien adaptées au maltose a une fréquence certaine. L'avancée des connaissances en biologie moléculaire sur la régulation des gènes d'un système donné permet d'expliquer comment, par recombinaison méïotique, les souches ayant les propriétés recherchées peuvent être construites avec une fréquence certaine. Par exemple, des travaux de biologie moléculaire ont montré que des mutations intervenant sur les gènes de régulation du système maltose MAL 13, MAL 23, MAL 43, MAL 64 conduisent à des souches ayant une expression constitutive des enzymes dégradant le maltose. Ces gènes mutés sont appelés MAL 13c, MAL 23c, MAL 43c, MAL 64c. Ces gènes de régulation ont en général un caractère dit "trans" qui donne à la mutation un caractère dominant et leur présence est souvent associée à une certaine dérépression par rapport au glucose. Si un évènement peut être prévu avec une fréquence certaine, le phénomène devient prévisible et reproductible.

L'invention a notamment pour but de fournir de nouvelles souches à large spectre et un procédé reproductible, essentiellement par hybridation, pour les construire, lesdites nouvelles souches et ledit procédé présentant des progrès importants par rapport à ceux de l'art antérieur.

Et la Société Demanderesse a eu le mérite de trouver, à l'issue de recherches approfondies, que ce but était atteint, en d'autres termes que de nouvelles souches de levure de panification à large spectre et caractérisées par les activités enzymatiques suivantes déterminées à l'aide des tests $T_1$, $T_2$ et $T_3$ décrits plus loin:

- activité maltose perméase après croissance de la levure sur milieu glucose, en l'absence de maltose (Test $T_1$): au moins 9 unités,
- activité maltase après croissance de la levure sur milieu glucose, en l'absence de maltose (Test $T_2$): au moins 80 unités et, de préférence, au moins 90 unités,
- activité invertase (Test $T_3$): moins de 10 unités et, de préférence, plus de 2 unités,

pouvaient être construites par mise en oeuvre d'un procédé consistant:

- à sélectionner des souches parentes

  . présentant de manière générale de bonnes propriétés de multiplication sur mélasses, d'assimilation de l'azote minéral, de préférence de résistance au séchage;
  . présentant sur pâtes normales ou, de préférence, sur pâtes sucrées des propriétés particulièrement remarquables;

- à faire sporuler les souches ainsi sélectionnées;
- à rechercher par des tests de sélection simples parmi les haploïdes (ségrégeants) ainsi obtenus, ceux qui auront de plus un potentiel élevé pour avoir des propriétés maximales sur les types de pâtes boulangères pour lesquels la souche parente n'est pas performante;
- à croiser ensemble les haploïdes à fort potentiel ainsi sélectionnés;
- à sélectionner les hybrides issus de ces croisements par les mêmes tests, puis par des tests de sélection plus complets.

Une variante à ce procédé consiste en complément, en cas d'une teneur en invertase trop élevée des haploïdes (ségrégeants) ou des hybrides obtenus, à abaisser cette teneur en invertase par les outils de la biologie moléculaire, notamment la disruption du ou des gènes codants pour l'invertase.

L'abaissement de la teneur en invertase des souches hybrides (diploïdes ou aneuploïdes) de levures de boulangerie ou des haploïdes (ségrégeants) par la biologie moléculaire, notamment par disruption d'un ou plusieurs gènes codant pour l'invertase (gène SUC) est une technique d'application générale, permettant d'obtenir des nouvelles souches selon l'invention.

Les tests utilisés pour mesurer les susdites unités enzymatiques et les définitions de ces unités sont indiqués ci-après.

L'unité maltose-perméase est exprimée en nanomoles de maltose entrant par minute dans 1 mg de matières sèches cellulaires levure.

Le dosage de la maltose-perméase, après culture sur milieu glucose, en l'absence de maltose (Test $T_1$), est dérivé du protocole de Serrano R., Eur. J. Biochem., 1977, 80, 97-102, et réalisé de la manière indiquée ci-après.

Les levures sont cultivées pendant 24 heures sur milieu YEG (Yeast extract 0,5%, glucose 2%, agar 3%). Les levures sont alors récoltées et lavées de manière à obtenir une suspension de levures contenant 4 à 5 mg de matières sèches levure par ml de tampon phosphate de Na 0,05 M (50 millimolaire), pH 6, suspension qui est mise à incuber au bain-marie à 20°C. On prépare par ailleurs une solution contenant d'une part, du maltose non radioactif et du maltose radioactif U - $C^{14}$ pour une concentration finale 1 M (molaire) et de 22 microcuries/ml (814 kilobecquerels/ml), et d'autre part du glucose 0,01 M (10 millimolaire). L'essai d'incorporation (entrée du maltose dans la cellule) est réalisé à 20°C pour limiter le métabolisme du maltose, en mélangeant 230 microlitres de la suspension de levure à 30 microlitres du mélange de sucres. La vitesse d'incorporation est estimée d'après la radioactivité intracellulaire pour des durées inférieures ou égales à 30 secondes. L'activité malto-perméase est exprimée en nanomoles de maltose entrant dans la cellule par minute et par mg de matières sèches.

Le dosage de la maltose-perméase après culture sur milieu maltose (Test $T'_1$) est réalisé, comme indiqué ci-dessus, si ce n'est que les levures sont cultivées pendant 24 heures sur milieu YEM où les 2% de glucose sont remplacés par 4% de maltose.

L'unité maltase est exprimée en nanomoles de PNP (paranitrophénol) libérées par minute et par mg de matières sèches levure.

Le dosage de la maltase après culture sur milieu glucose, en l'absence de maltose (Test $T_2$), est réalisé de la manière indiquée ci-après.

Les levures sont cultivées sur milieu YEG pendant 24 heures comme pour le dosage de la maltose-perméase. Les levures sont récoltées et lavées de manière à obtenir une suspension contenant 10 à 20 milligrammes de matières sèches levure par millilitre, les levures sont perméabilisées par le chloroforme et, pour ce faire, on ajoute 0,3 ml de chloroforme à 2 ml de suspension; on agite l'ensemble pendant 30 minutes à 30°C, puis on dilue à 20 ml avec de l'eau à 4°C. Ensuite, la maltase est dosée par la méthode de Halvorson H. et Elias E.L. Biochim. Biophys. Acta, 1958, 30, 28.

Le dosage de la maltase après culture sur milieu maltose (Test $T'_2$) est réalisé comme indiqué ci-dessus si ce n'est que les levures sont cultivées pendant 24 heures sur milieu YEM où les 2% de glucose sont remplacés par 4% de maltose.

L'unité invertase est définie comme étant la production d'une micromole de sucres réducteurs, correspondant en l'occurrence à une demi-micromole de saccharose inverti, en 5 minutes par mg de matières sèches de levures, à 30°C et à pH 4,7, sans plasmolyse de la levure; pour la doser (Test $T_3$), on met en présence une quantité connue, de l'ordre de quelques dizièmes de mg, de matières sèches levure et du saccharose à concentration 0,1 molaire dans un tube à essais en milieu tamponné (tampon acétate à pH 4,7) placé dans un bain-marie à 30°C. Au bout de 5 minutes, la réaction d'inversion du saccharose est bloquée par ajout du réactif au dinitrosalycilate de soude qui sert à doser les sucres réducteurs formés par réaction colorimétrique.

Les valeurs indiquées plus haut des susdites activités enzymatiques caractéristiques des souches selon l'invention montrent que les souches en question:

. sont parfaitement adaptées au maltose, c'est-à-dire ont des activités constitutives maltose-perméase et maltase en milieu glucose qui ne sont pas limitantes,

. ont une activité invertase suffisamment importante pour ne pas être limitante et suffisamment faible pour ne pas créer rapidement une augmentation de la pression osmotique due à l'hydrolyse du saccharose.

Les susdites activités enzymatiques sont, dans le cas des nouvelles souches conformes à l'invention, des conditions nécessaires mais non suffisantes à elles seules.

Il faut que les souches en question aient de plus

- un bon rendement de multiplication,
- une bonne assimilation de l'azote,
- une bonne activité fermentative du glucose, étant entendu que le maltose ou le saccharose sont toujours fermentés sous forme de glucose ou de fructose,
- une bonne résistance au séchage,

propriétés qui sont obtenues grâce à la sélection conforme à l'invention des souches parentes.

Le nouveau produit industriel constitué par la levure de panification préparée sous forme fraîche à l'aide des susdites nouvelles souches pouvant être obtenues par le procédé conforme à l'invention, est caractérisé par le fait que, simultanément dans les tests A décrits ci-après, elle présente les caractéristiques suivantes:

- dans le test $A_1$ sur 2 heures, elle donne un dégagement gazeux compris entre 170 et 230 ml,
- dans le test $A_5$ sur 2 heures, elle donne un dégagement gazeux compris entre 130 et 180 ml, de préférence entre 140 et 180 ml,
- dans le test $A_6$ sur 2 heures, elle donne un dégagement gazeux compris entre 170 et 230 ml, de préférence entre 180 et 250 ml.

Le nouveau produit industriel constitué par la levure sèche de panification préparée à l'aide des susdites nouvelles souches pouvant être obtenues par le procédé conforme à l'invention, est caractérisé par le fait que, simultanément dans les tests A' décrits ci-après, elle présente les caractéristiques suivantes:

- dans le test $A'_1$ sur 2 heures, elle donne un dégagement gazeux compris entre 120 et 145 ml,
- dans le test $A'_5$ sur 2 heures, elle donne un dégagement gazeux compris entre 95 et 130 ml, de préférence entre 100 et 130ml,
- dans le test $A'_6$ sur 2 heures, elle donne un dégagement gazeux compris entre 135 et 190 ml, de préférence entre 140 et 190 ml, et encore plus préférentiellement entre 150 et 190 ml.

Ces valeurs caractéristiques tant pour la levure fraîche comprimée selon l'invention que pour la levure sèche active selon l'invention, montrent qu'il s'agit d'une levure qui est aussi rapide sur pâte boulangère sans sucre ou avec peu de sucre que les meilleures levures produites pour fermenter des pâtes sans sucre ou peu sucrées, et qui est également sur pâte à plus de 15% de sucre au moins aussi performante que les levures spécialisées pour la fermentation de ces pâtes sucrées, alors que ces propriétés ne se retrouvent jamais simultanément dans les souches actuellement connues.

Les tests A et A' utilisés par la Demanderesse pour caractériser les susdites levures sont réalisés à l'aide du fermentomètre de Burrows et Harrison décrit dans le "Journal of the Institute of Brewing", vol. LXV, No.1, January-February 1959 et sont exactement définis de la manière suivante:

Test $A_1$ (levures comprimées fraîches).

A 20 g de farine incubée à 30°C, on ajoute un poids de levure comprimée correspondant à 160 mg de matières sèches, cette levure étant délayée dans 15 ml d'eau contenant 27 g de NaCl par litre et 4 g de $SO_4(NH_4)_2$ par litre; on malaxe à l'aide d'une spatule pendant 40 secondes, de manière à obtenir une pâte que l'on place au bain-marie réglé à 30°C; treize minutes après le début du malaxage, le récipient contenant la pâte est fermé hermétiquement; la quantité totale de gaz produit est mesurée après 60, puis 120 minutes; cette quantité est exprimée en ml à 30°C et sous 760 mm de Hg.

Pour toutes les levures susceptibles de donner en 120 minutes un dégagement gazeux égal ou supérieur à 150 ml de $CO_2$, la quantité de sucres fermentescibles apportée uniquement par la farine est limitante; en conséquence, le test est modifié de la manière suivante: on ajoute un poids de levure correspondant à 106 mg de matières sèches levure, au lieu de 160 mg, et la lecture de la quantité de gaz produite est par convention multipliée par 1,5.

Test A'$_1$ (levures sèches).

Identique à l'essai A$_1$, mais préalablement au malaxage, on réhydrate en 15 minutes les 160 mg de matières sèches levure qui se présentent sous forme de levure sèche active dans de l'eau distillée, à 38°C; on utilise à cet effet 40% du volume d'eau d'hydratation mis en oeuvre; le complément en eau, additionné de 405 mg de NaCl, est ajouté à l'issue des 15 minutes de réhydratation.

Test A$_3$ (levures comprimées fraîches).

Essai identique à l'essai A'$_1$, mais on ajoute à la farine 2 g de saccharose; la quantité totale de gaz produit est mesurée après 60 minutes.

Test A'$_3$ (levures sèches).

Essai identique à l'essai A'$_1$, mais on ajoute à la farine 2 g de saccharose; la quantité totale de gaz produit est mesurée après 60 minutes.

Test A$_4$ (levures comprimées fraîches).

Essai identique à l'essai A'$_1$, mais on ajoute à la farine 5,5 g de saccharose; la quantité totale de gaz produit est mesurée après 60 minutes.

Test A'$_4$ (levures sèches).

Essai identique à l'essai A'$_1$, mais on ajoute à la farine 5,5 g de saccharose; la quantité totale de gaz produit est mesurée après 60 minutes.

Test A$_5$ (levures comprimées fraîches).

Essai identique à l'essai A$_1$, mais on ajoute à la farine 4 g de saccharose; la quantité totale de gaz produit est mesurée après 60 minutes et 120 minutes.

Test A'$_5$ (levures sèches).

Essai identique à l'essai A'$_1$, mais on ajoute à la farine 4 g de saccharose; la quantité totale de gaz produit est mesurée après 60 minutes et 120 minutes.

Test A$_6$ (levures comprimées fraîches).

A 25 g de farine incubée à 30°C, on ajoute 6,5 g de sucre glace et un poids de levure comprimée correspondant à 320 mg de matières sèches, ensuite on procède comme pour l'essai A$_1$ et la quantité totale de gaz produit est mesurée après 60 et 120 minutes.

Test A'$_6$ (levures sèches).

Essai identique à l'essai A$_6$, en procédant pour réhydrater les 320 mg de matières sèches levure sous forme levure sèche active comme dans l'essai A'$_1$.

Ceci étant, pour construire les nouvelles souches, conformément à l'invention on procède comme suit.

Dans une première étape, on sélectionne les souches parentes. De préférence, on utilise pour cette sélection les meilleures souches déjà connues comme levures de panification, ces souches ayant déjà un certain nombre de propriétés de base indispensables; la plupart de ces souches peuvent être obtenues auprès des centres de collection internationaux et notamment à l'ATCC (American Type Culture Collection), où elles sont déposées.

Elles peuvent être classées en deux grands groupes, à savoir:

- les souches très rapides sur pâte sans sucre, qui donnent dans le test A$_1$ en 2 heures des levures fraîches pouvant avoir une activité de 200 ml de $CO_2$ ou plus, mais qui parallèlement donnent des activités en général inférieures à 70 ml de $CO_2$ dans le test A$_6$ en 2 heures; ces souches très rapides, très bien adaptées au maltose ont des activités maltose-perméase constitutive et maltase constitutive élevées; elles se caractérisent par ailleurs par une activité invertase élevée;

- les souches très bien adaptées aux pâtes sucrées qui ont une activité dépassant nettement 150 ml dans le test A$_6$ en 2 heures mais dont les activités dans le test A$_1$ en 2 heures ne sont que de l'ordre de 100 ml de $CO_2$; ces souches très bien adaptées aux pâtes sucrées ont des activités maltose-perméase constitutive, maltase constitutive et invertase faibles, voire très faibles.

De préférence, on utilise des souches parentes-du deuxième groupe, et on sélectionne celles

- qui sont performantes dans le test A$_6$, c'est-à-dire qui donnent lieu à un dégagement gazeux d'au moins 160 ml en 2 heures dans ce test,

- qui donnent un rendement acceptable sur mélasses y compris à des taux de multiplication horaire élevés de l'ordre de 1,20,

- qui assimilent aisément l'azote et sont susceptibles de donner des levures de panification, dans des conditions courantes de culture, avec des teneurs en azote sur matières sèches de l'ordre de 9.

De préférence, ces souches parentes ainsi sélectionnées sont, de plus, stables au séchage.

On a constaté qu'une proportion significative des souches osmotolérantes utilisées industriellement présentent ces propriétés, ce grâce à quoi il n'y a aucune difficulté à sélectionner des souches parentes convenables.

Dans le cas des souches parentes du premier groupe, on sélectionne celles

- qui, d'une part, donnent au moins 170 ml et de préférence au moins 180 ml en 2 heures dans le test $A_1$, qui donnent un rendement acceptable sur mélasses y compris à des taux de multiplication horaire élevés de l'ordre de 1,20, et qui assimilent aisément l'azote,
- qui, d'autre part, ne sont pas trop osmosensibles, point qui est étudié en remplaçant dans les tests $A_5$ et $A_6$ le saccharose par une même quantité de glucose, étant entendu qu'on retient les souches donnant au moins 70 ml de $CO_2$, de préférence au moins 80 ml et, plus préférentiellement, au moins 90 ml en 2 heures dans le test $A_5$ où le saccharose a été remplacé par une même quantité de glucose.

L'osmosensibilité des souches peut aussi être étudiée en ajoutant à la farine du test $A_1$ sur 2 heures, d'une part, 1 g de glucose (Test $A_9$) et, d'autre part, 1 g de glucose et 3 g de sorbitol (Test $A_{10}$) et en faisant le rapport Test $A_{10}$/ Test $A_9$. Dans ce test, on retiendra toutes les souches pour lesquelles ce rapport est au moins égal à 0,40 et, de préférence, celles pour lesquelles il est au moins égal à 0,45.

Dans une deuxième étape, on fait sporuler les souches parentes ainsi sélectionnées.

Conformément à l'invention, on sélectionne parmi les haploïdes obtenus, ceux qui simultanément:

. se colorent en bleu lorsqu'ils sont cultivés sur un milieu à base de glucose comme le milieu YEG (Yeast Extract 0,5%, glucose 2%, agar 3%) en présence de 5-bromo-4-chloro-3-indolyl-alpha-D-glucopyranoside appelé ci-après X alpha-glu, qui est un analogue du maltose;

. ont un taux d'invertase inférieur à 10 unités et, de préférence, supérieur à 2 unités.

Le susdit test de coloration bleue réalisé sur boîte de Pétri est de mise en oeuvre très simple. Les souches à tester sont repiquées en quadrillage sur un milieu à base de glucose comme, par exemple, le milieu YEG contenant le X alpha-glu (40 microlitres par boîte de Pétri d'une solution à 2% de X alpha-glu dans le diméthylformamide). Si le X alpha-glu a pu pénétrer dans la cellule, et si la maltase interne à la levure est présente sous forme constitutive et est non réprimée par le glucose, la maltase coupe la liaison alpha-glucoside de ce composé et libère un radical de couleur bleue intense, qui précipite.

Après croissance, les colonies correspondant aux souches déréprimées pour le système maltose présentent ladite coloration bleue intense dans le test en question. Ce test de coloration bleue sur boîte de Pétri est très rapide et permet de tester rapidement d'une façon simple de très nombreux haploïdes. Il est particulièrement intéressant pour la recherche des haploïdes déréprimés pour le système maltose parmi les ségrégeants obtenus à partir de souches sélectionnées pour les performances dans le test $A_6$, mais peu actifs sur pâte sans sucre en raison de leur non adaptation au maltose. Il permet de sélectionner d'une manière étonnamment simple et sûre parmi les haploïdes issus de souches parentes qui ne sont pas du tout adaptées au maltose, la proportion de ségrégeants (haploïdes) --en l'occurrence quelques pour cent-- qui ont des activités maltose-perméase et maltase constitutives se situant à des niveaux nécessaires et suffisants pour pouvoir conduire par hybridation à des souches très bien adaptées au maltose et donc très rapides sur pâte sans sucre.

La mesure du taux d'invertase est d'une mise en oeuvre un peu plus lourde. Dans le cas, notamment où l'on cherche à sélectionner des ségrégeants pauvres en invertase issus de souches parentes sélectionnées en raison de leurs performances dans le test $A_1$ (dégagement d'au moins 170 ml et de préférence au moins 180 ml sur 2 heures), on peut avoir recours à un test colorimétrique semi-quantitatif, dérivé du test mis au point par TRUMBLY, Journal of Bacteriology, 1986, 166, p. 1123.

Pour ce faire, on peut utiliser une plaque de microtitration. Dans chaque cupule de ladite plaque, on introduit 20 microlitres d'eau, puis on introduit une quantité de la colonie à tester correspondant au volume d'une tête d'épingle, c'est-à-dire environ 0,05 mg de matières sèches levure (exemptes du milieu de culture) et enfin on ajoute 50 microlitres d'une solution de saccharose à 6% dans du tampon acétate de sodium 0,01 M (10 millimolaire), à pH 4,6.

On met ensuite la plaque de microtitration à incuber pendant 5 minutes à 30°C au bain-marie. On ajoute alors 50 microlitres de chlorure de triphényl-tétrazolium à 0,1% dans NaOH 0,5 molaire. On incube à nouveau pendant 2 minutes à 50°C au bain-marie.

Il se développe une coloration rouge d'autant plus intense que le taux d'invertase du ségrégeant (haploïde) est élevé. Expérimentalement, on peut de manière simple, par ce test de coloration, sélectionner les haploïdes ayant moins de 20 unités invertase et, de préférence, moins de 10 unités invertase.

La sélection ainsi effectuée des haploïdes doit être confirmée par la mesure de leur activité enzymatique. Lesdits haploïdes devront présenter simultanément les activités suivantes:

- maltose-perméase après croissance sur glucose: au moins 3 unités et, de préférence, au moins 7 unités,
- maltase après croissance sur glucose: au moins 40 unités et, de préférence, au moins 80 unités,
- invertase: moins de 10 unités et, de préférence, entre 2 et 10 unités.

De préférence, ces haploïdes présenteront également les activités suivantes:

- maltose-perméase après culture sur maltose: au moins 12 unités,
- maltase après culture sur maltose: au moins 200 unités.

Dans l'étape suivante, on procède à l'hybridation de l'ensemble des haploïdes sélectionnés, c'est-à-dire des haploïdes qui répondent à l'ensemble des critères définis ci-dessus.

On signale que la sporulation des souches de départ, l'obtention des haploïdes et la conjugaison de ces haploïdes sont effectuées par mise en oeuvre des techniques décrites dans le chapitre 7 "Sporulation and Hybridization of Yeast" par R.R. Fowell, de l'ouvrage "The Yeasts", volume 1, édité par A.H. Rose and J.S. Harrison, 1969 - Academic Press. La technique d'hybridation retenue est la technique de la conjugaison en masse (mass-mating).

On sélectionne ensuite, à partir de tous les hybrides ainsi obtenus, les souches conformes à l'invention en ayant recours aux mêmes tests que pour la sélection des haploïdes, à savoir:

- développement d'une culture bleue après étalement sur boîte de Pétri YEG en présence de X alpha-glu,
- non développement d'une coloration rose dans le test pour déterminer le taux d'invertase d'après Trumbly,
- maltose-perméase après culture de la levure sur milieu glucose, en l'absence de maltose, au moins 9 unités,
- maltose-perméase après culture de la levure sur milieu maltose, au moins 12 unités,
- maltase après culture de la levure sur milieu glucose, en l'absence de maltose, au moins 50 unités, de préférence au moins 80 unités et, encore de préférence, au moins 90 unités,
- maltase après culture de la levure sur milieu maltose, au moins 200 unités,
- invertase: moins de 10 unités, de préférence plus de 2 unités,

et on affine la sélection en ayant recours à des tests de culture selon des méthodes usuelles, par un tri supplémentaire:

- en fonction du rendement,
- en fonction de l'assimilation d'azote,
- en fonction des activités dans les tests $A_1$ et $A_6$,
- en fonction de la résistance au séchage.

De manière à augmenter les croisements possibles et leurs performances, il est intéressant de rechercher expérimentalement parmi les haploïdes sélectionnés comme indiqué plus haut et objet des croisements définis ci-dessus:

. ceux qui, de manière dominante, même après croisement avec des haploïdes ayant une invertase supérieure à 10, donneront des souches ayant une invertase inférieure à 10;
. ceux qui, de manière dominante, apporteront après culture sur glucose en l'absence de maltose, des activités maltose-perméase et maltase élevées.

Comme l'expérience a montré qu'il était moins fréquent d'obtenir des haploïdes convenables à partir des souches définies ci-dessus comme les souches parentes du premier groupe, celles-ci donnant une proportion importante d'haploïdes ayant des activités maltose-perméase constitutive et maltase constitutive élevées, mais aussi une activité invertase trop élevée, il a été constaté qu'il était possible d'abaisser cette activité invertase par les outils de la biologie moléculaire, par exemple en disruptant un ou plusieurs gènes SUC. Cette technique peut également être appliquée aux hybrides après croisement.

Pour préparer la levure fraîche de panification conforme à l'invention, on cultive les susdites souches selon les méthodes habituelles mais avec un taux de multiplication horaire moyen de l'ordre de 1,18 à 1,20 de manière à obtenir des levures comprimées à environ 30% de matières sèches ayant moins de 10% de bourgeons, des teneurs en azote sur matières sèches de l'ordre de 8,7 à 9 et des teneurs en $P_2O_5$ sur matières sèches de l'ordre de 3%.

La levure fraîche comprimée ainsi obtenue est caractérisée par le fait que:

dans le test $A_1$ sur 2 heures, elle donne un dégagement gazeux compris entre 170 et 230 ml, de préférence entre 190 et 230 ml,
dans le test $A_5$ sur 2 heures, elle donne un dégagement gazeux compris entre 130 et 170 ml, de préférence entre 140 et 180 ml, plus préférentiellement encore entre 150 et 180 ml,
dans le test $A_6$ sur 2 heures, elle donne un dégagement gazeux compris entre 170 et 230 ml, de préférence entre 180 et 250 ml, plus préférentiellement encore entre 200 et 250 ml.

Pour préparer la levure active sèche conforme à l'invention, les nouvelles souches obtenues sont cultivées selon les méthodes habituelles, en milieu concentré, c'est-à-dire dans un milieu où le poids total du moût en fin de culture par rapport à la quantité de mélasses coulée est de l'ordre de 4,7 à 5,5 avec un taux de multiplication horaire moyen de l'ordre de 1,17 à 1,18, de manière à obtenir de nouvelles levures comprimées à 30-35% de matières sèches:

- ayant une quantité de bourgeons inférieure à 5%,
- une teneur en protéines sur matières sèches de l'ordre de 7,9 à 8,3,
- une teneur en $P_2O_5$ sur matières sèches de l'ordre de 2,7 à 2,8,
- une teneur en tréhalose sur matières sèches de l'ordre de 10 à 13%, de préférence de l'ordre de 12 à 13%.

Cette levure fraîche est séchée par un séchage rapide ménageant en présence d'un émulsifiant, par exemple en présence de 1,5% de monostéarate de sorbitan.
La nouvelle levure sèche ainsi obtenue est caractérisée par le fait que:

- dans le test $A'_1$ sur 2 heures, elle donne un dégagement gazeux compris entre 120 et 145 ml, de préférence entre 130 et 145 ml et, plus préférentiellent encore, entre 135 et 145 ml,
- dans le test $A'_5$ sur 2 heures, elle donne un dégagement gazeux compris entre 95 et 130 ml, de préférence entre 100 et 130 ml, plus préférentiellement encore entre 110 et 130 ml, et
- dans le test $A'_6$ sur 2 heures, elle donne un dégagement gazeux compris entre 135 et 190 ml, de préférence entre 140 et 190 ml et, plus préférentiellement encore, entre 150 et 190 ml.

Ceci étant, l'invention est illustrée par les exemples suivants.

## <u>EXEMPLE 1</u>

On isole les souches de levures fraîches et de levures sèches osmotolérantes commercialisées en Europe, aux Etats-Unis d'Amérique et au Japon.
On les teste dans le cadre d'une culture reproduisant des conditions industrielles, en milieu concentré, à un taux de multiplication moyen de l'ordre de 1,18 conduisant:

- à des levures pressées à 8,8-9% en poids d'azote sur matières sèches,
- à des levures sèches à 8% en poids d'azote sur matières sèches.

On retient les souches qui simultanément

- donnent un rendement en matières sèches levures sur mélasse de betteraves à 50 degrés Clerget (mesure du saccharose par double polarisation) d'au moins 23% et, de préférence, d'au moins 25%,
- donnent des levures pressées conduisant, dans le test $A_6$, à au moins 160 ml de gaz carbonique,
- donnent des levures sèches conduisant, dans le test $A'_6$, à au moins 120 ml,
- ont un taux d'invertase inférieur à 10 unités.

On fait sporuler les souches retenues, qui ne sont pas adaptées au maltose. Aucune souche connue répondant aux critères énumérés ci-dessus, ne permet d'obtenir des levures fraîches comprimées donnant significativement de 100 ml à 110 ml en 2 heures dans le test $A_1$. On recherche les haploïdes donnant des colonies bleues dans le test sur boîte de Pétri avec milieu YEG (Yeast Extract Glucose) et X alpha-glu.
On vérifie que les haploïdes donnant des colonies bleues répondent simultanément à tous les critères suivants:

- activité maltose-perméase après culture sur milieu glucose, au moins 3 unités et, de préférence, au moins 7 unités,
- activité maltose-perméase après culture sur milieu maltose, au moins 12 unités,
- activité maltase après culture sur milieu glucose, au moins 40 unités et, de préférence, au moins 80 unités,
- activité maltase après culture sur milieu maltose, au moins 200 unités,

- activité invertase, moins de 10 unités.

On a ainsi obtenu une vingtaine d'haploïdes répondant simultanément à l'ensemble de ces critères.

A titre d'exemple, on signale que trois haploïdes ont été déposés dans la Collection Nationale de Cultures de Microorganismes (CNCM) à l'Institut Pasteur à Paris, 25 rue du Docteur Roux, 75724 PARIS Cédex 15. Ces haploïdes sont désignés dans les cahiers de laboratoire de la Demanderesse sous les numéros:

HO 37 de signe de conjugaison a déposé dans la CNCM sous le numéro I-1076
HCT 14 de signe de conjugaison alpha déposé dans la CNCM sous le numéro I-1075
HCT 44 de signe de conjugaison alpha déposé dans la CNCM sous le numéro I-1074.

Ces haploïdes présentent les activités enzymatiques suivantes:

| | maltose-perméase | maltose-perméase | maltase | maltase | invertase |
|---|---|---|---|---|---|
| | après culture sur milieu | | | | |
| | glucose | maltose | glucose | maltose | |
| HO 37 | 3,5 | 26 | 115 | 230 | 7 |
| HCT 14 | 8,0 | 22 | 45 | 240 | 6 |
| HCT 44 | 12,0 | 15 | 135 | 340 | 2 |

## EXEMPLE 2

On croise ensemble les haploïdes sélectionnés selon l'exemple 1. On sélectionne les souches répondant aux critères suivants.

Dans un premier temps, on sélectionne les souches ayant simultanément les activités enzymatiques suivantes:

- maltose-perméase après culture sur milieu glucose, au moins 9 unités,
- maltose-perméase après culture sur milieu maltose, au moins 12 unités,
- maltase après culture sur milieu glucose, au moins 90 unités,
- maltase après culture sur milieu maltose, au moins 200 unités et, de préférence, au moins 230 unités,
- invertase: moins de 10 unités et, de préférence, plus de 2 unités.

Dans un second temps, on vérifie par des tests de culture les performances des souches ainsi sélectionnées. On peut faire des tests semi-anaérobies de laboratoire donnant une récolte suffisante pour permettre des mesures d'activité fermentative; on peut également faire des cultures aérobies en volumes réduits. Ces tests ont le mérite de pouvoir être effectués rapidement sur un grand nombre de souches. Ils permettent le classement des souches obtenues, la vérification qu'elles ont bien les performances recherchées, par rapport à des souches témoins.

Dans un dernier temps, on vérifie que les souches ayant passé le deuxième crible donnent une nouvelle levure de panification selon l'invention dans le cadre d'essais pilotes reproduisant aussi exactement que possible les conditions de la production industrielle, c'est-à-dire:

- les cycles de fabrication successifs,
- la concentration du milieu de culture illustré par exemple par le ratio

$$\frac{\text{poids final du moût levuré dans le cuve de fermentation}}{\text{mélasse totale à 50\% de saccharose}}$$

qui a été choisi dans le cadre des essais comme étant de l'ordre de 5,
- les conditions d'aération, le taux d'alcool en cuve.

Dans le cadre de cet exemple, trois souches ont été sélectionnées comme donnant des produits nouveaux particulièrement intéressants, c'est-à-dire la nouvelle levure fraîche comprimée et la nouvelle levure sèche selon l'invention. Ces trois souches ne sont que des exemples, d'autres souches équivalentes ont été et pourront être obtenues.

Les trois souches sont désignées dans les cahiers de laboratoire de la Demanderesse et déposées à la Collection Nationale de Cultures de Microorganismes, 25 rue du Docteur Roux, 75724 PARIS Cédex 15, sous les numéros:

n° 3493 déposée dans la CNCM sous le n° I-1073
n° 3603 déposée dans la CNCM sous le n° I-1072
n° 3608 déposée dans la CNCM sous le n° I-1071.

Ces trois souches ont les activités enzymatiques suivantes, qui sont comparées avec les activités enzymatiques de quelques souches-types.

| | maltose-perméase | maltose-perméase | maltase | maltase | invertase |
|---|---|---|---|---|---|
| | après culture sur milieu | | | | |
| | glucose | maltose | glucose | maltose | |
| 3493 | 14 | 21 | 130 | 240 | 5 |
| 3603 | 16 | 27 | 230 | 330 | 4 |
| 3608 | 16 | 36 | 90 | 250 | 8 |
| Souche isolée d'une levure très rapide sur pâte normale commercialisée en Grande-Bretagne | 9 | 12 | 90 | 230 | 100 |
| Souche isolée d'une levure très osmotolérante commercialisée au Japon | 1 | 14 | 20 | 130 | 1,5 |
| NCYC 995 | 8 | 16 | 110 | 290 | 40 |
| NCYC 996 | | | | 24 | 20 |

Les trois haploïdes HO 37, HCT 14, HCT 44, les trois souches 3493, 3603, 3608 présentent les caryotypes suivants par électrophorèse à champs pulsés:

Sur ce schéma, ont été ajoutées à titre de témoins les souches de levures de boulangerie NCYC 995 et NCYC 996, objet du brevet U.S. n° 4.396.632 ainsi que deux souches témoins de laboratoires :

Lα3 :      souche haploïde témoin de laboratoire,

YPH80 :   Yeast Chromosome PFG Marker, vendu par New England BIOLABS, 32 Tozer Road, Beverly, MA 01915/USA.

## EXEMPLE 3

Les trois nouvelles souches 3493, 3603, 3608 déposées dans la CNCM sous les n° I-1073, I-1072, I-1071, la souche isolée à partir de la meilleure levure fraîche pour ses performances sur pâte normale, trouvée sur le marché britannique en 1990, désignée ci-après comme souche rapide britannique, la souche isolée à partir de la meilleure levure pour ses performances sur pâtes très sucrées, trouvée sur le marché japonais en 1990, désignée ci-après comme souche osmotolérante japonaise, ont été cultivées de la manière suivante.

A l'exception de l'emploi de procédés et matériels spécifiés ci-après, les souches ont été propagées en plusieurs stades de multiplication aérobie, la levure fraîche a été récoltée, lavée, filtrée à l'aide de matériels classiques employés en levurerie et selon les procédés de fabrication habituels comme les matériels et les procédés décrits dans l'ouvrage "Yeast Technology" de Gerald Reed and Henry J. Peppler (1973), The Avi Publishing Company Inc. ou dans le chapître "Production of Baker's Yeast", Gerald Reed, publié par Prescott and Dunn's Industrial Microbiology, 4ème Edition, édité par Gerald Reed, The Avi Publishing Co. Inc., second printing 1983.

Il est particulièrement veillé à ce que tous les nutriments nécessaires en petites quantités à la levure, minéraux (macroéléments et oligoéléments), vitamines (biotine, vitamines de groupe B) soient présents au moins dans les quantités les plus grandes recommandées dans les ouvrages de référence cités ci-dessus. De manière générale, ces essais sont menés comme indiqué dans les précédents brevets de la Demanderesse, cités ci-dessus. Il est notamment veillé à obtenir des levures bien lavées et à refroidir rapidement la crème et les levures filtrées à 2°C.

Le dernier stade de multiplication de la levure conduisant à une levure fraîche comprimée très active est mené plus spécifiquement de la manière suivante:

. dilution du milieu de culture en fin de multiplication commerciale:

$$\frac{\text{Poids du moût levuré dans la cuve}}{\text{Quantité de mélasse à 50\% de sucres totaux exprimés en saccharose}} = 5{,}2$$

De préférence, ces essais sont menés avec un mélange de 90% de mélasses de betterave, 10% de mélasses de canne, ces deux mélasses devant être de bonne qualité, c'est-à-dire de bonne pureté et ne pas contenir d'inhibiteurs ou de substances toxiques pour les levures. Il doit en effet être particulièrement vérifié par des essais de cultures témoins que les mélasses ne contiennent pas d'additifs toxiques parfois ajoutés lors du travail d'extraction et de purification du sucre en sucrerie. Le sucre des mélasses de betterave est mesuré par la méthode Clerget (détermination du saccharose par double polarisation), le sucre des mélasses de canne est déterminé par mesure enzymatique du saccharose, glucose et fructose réellement présents et l'ensemble des ces sucres est calculé en équivalent saccharose;

. taux de multiplication horaire moyen sur le dernier cycle de multiplication de 14 heures: 1,18 à 1,20.

. taux maximum de bourgeons de la levure récoltée: 10%.

. taux azote/matières sèches levure récoltée: 9% (8,6 à 9,2).

. taux $P_2O_5$/matières sèches levure récoltée: 3%.

Ces essais ont donné des levures fraîches de panification à environ 30% de matières sèches ayant les caractéristiques suivantes:

| | Test $A_1$ 2 heures | Test $A_5$ 2 heures | Test $A_6$ 2 heures |
|---|---|---|---|
| Souche 3493 | 198 | 154 | 215 |
| Souche 3603 | 193 | 150 | 223 |
| Souche 3608 | 210 | 165 | 235 |

(suite)

|  | Test $A_1$ 2 heures | Test $A_5$ 2 heures | Test $A_6$ 2 heures |
|---|---|---|---|
| Souche NCYC 995 | 180 | 110 | 135 |
| Souche rapide britannique | 210 | 90 | 55 |
| Souche osmotolérante japonaise | 110 | 135 | 198 |

La souche NCYC 995 était la meilleure souche connue jusqu'alors pour combiner des propriétés de rapidité sur pâte normale (sans sucre) et sur pâtes sucrées. Dans le tableau ci-dessus, elle est à 8,6 d'azote sur matières sèches, ce qui est proche du maximum pour sa production industrielle.

## EXEMPLE 4

On procède comme dans l'exemple précédent avec les mêmes souches dans le but d'obtenir des levures sèches actives. Les conditions générales de culture restent substantiellement les mêmes. Le taux de multiplication horaire moyen est de l'ordre de 1,18. Les conditions particulières de la culture, notamment les coulées de mélasses et d'ingrédients sont adaptées de manière à viser la composition suivante pour la levure récoltée destinée au séchage:

| azote sur matières sèches | valeur optimum ( 8%) |
|---|---|
| $P_2O_5$ sur matières sèches | 2,7 à 2,8% |
| tréhalose sur matières sèches | 12% |
| taux maximum de bourgeons de la levure récoltée | 5%. |

Pour la production des levures fraîches et sèches, il existe pour chaque souche une teneur optimum en azote sur matière sèche, en fonction des autres propriétés désirées pour la levure. A partir d'une certaine teneur en protéines, les gains d'activité fermentative deviennent faibles, voire très faibles ou même nuls. Cette notion est très importante pour les levures sèches. A partir d'une certaine teneur en protéines, les gains d'activité fermentative deviennent inférieurs à l'augmentation des pertes au séchage liées à cette teneur en protéines. Pour les nouvelles souches, l'optimum de composition en azote sur matières sèches pour la production de levures sèches est de l'ordre de 8 à 8,3%.

A cette levure, on ajoute une fine émulsion à base d'un émulsifiant comme le monostéarate de sorbitan, celui-ci étant ajouté à raison de 1,5% de matières sèches levure. L'émulsion peut également contenir un agent épaississant. On extrude la composition obtenue à travers une grille de largeur de maille 0,5 à 1 mm et on sèche ladite levure à au moins 92%, de préférence au moins 94% de matières sèches par un séchage rapide particulièrement ménageant, c'est-à-dire un séchage de moins d'une heure où la température de la levure ne dépasse pas 30°C en début du séchage et 40°C en fin de séchage. De préférence, on a ajouté une fine émulsion constituée de monostéarate de sorbitan à raison de 1,5% de la matière sèche levure et de carboxyméthylcellulose à raison de 0,5% de la matière sèche levure et on a séché rapidement la levure par fluidisation dans un courant d'air sec et chaud, comme indiqué page 6 du brevet européen 0 008 554. La levure sèche à la sortie du fluidiseur est rapidement refroidie et emballée dans des emballages hermétiques sous atmosphère inerte, c'est-à-dire sous un gaz neutre (azote, gaz carbonique) contenant moins de 1% d'oxygène. On a ainsi obtenu des levures sèches à environ 95% de matières sèches.

| Souche | N/MS | Levures fraîches à 30-35% matières sèches | | | Levures sèches à 95% matières sèches | | |
|---|---|---|---|---|---|---|---|
| | | A1 2 heures | A5 2 heures | A6 2 heures | A'1 2 heures | A'5 2 heures | A'6 2 heures |
| Souche 3493 | 8-8,2 | 172 | 140 | 200 | 137 | 113 | 160 |
| Souche 3603 | 8 | 182 | 147 | 220 | 122 | 105 | 150 |
| Souche 3608 | 8 | 193 | 151 | 226 | 135 | 115 | 170 |

(suite)

| Souche | N/MS | Levures fraîches à 30-35% matières sèches | | | Levures sèches à 95% matières sèches | | |
|---|---|---|---|---|---|---|---|
| | | A1<br>2 heures | A5<br>2 heures | A6<br>2 heures | A'1<br>2 heures | A'5<br>2 heures | A'6<br>2 heures |
| Souche NCYC 995 | 7,5-7,8 | 154 | 100 | 125 | 131 | 82 | 100 |
| Souche NCYC 996 | 7,2-7,5 | 105 | 125 | 165 | 91 | 100 | 130 |
| Souche rapide britannique | 8,2 | 185 | 90 | 70 | 125 | 60 | 40 |
| Souche osmotolérante japonaise | 8 | 95 | 120 | 180 | 70 | 95 | 145 |

## EXEMPLE 5

Disruption d'un gène codant pour l'invertase dans des souches haploïdes (ségrégeants) ou des hybrides (diploïdes ou aneuploïdes) ayant des activités maltose-perméase constitutive, maltase constitutive élevées, mais aussi des activités invertase élevées.

**1 - Technique de disruption d'un gène codant pour l'invertase dans une souche de Saccharomyces cerevisiae.**

1.1 Clonage de la partie codante du gène *SUC 2* par PCR (Polymerase Chain Reaction) ou amplification génique.

La séquence du gène *SUC 2* est décrite dans "Nucleotide sequence of the yeast *SUC 2* gene for invertase", Taussig, R. and Carlson, M., Nucleic Acids Res. (1983) 6, 1944-1954. Elle se trouve aussi dans les banques de données de séquences d'acides nucléiques, européenne ou américaine (EMBL et GENBANK).

L'amplification de la partie codante du gène *SUC 2* par PCR a été réalisée par le kit Gene Amp de la Société Perkin Elmer (avec Ampli Taq clonée) sur matériel DNA Thermal-Cycler (Perkin Elmer, 1 avenue Franklin, Montigny-le-Bretonneux, 78054 St Quentin en Yvelines Cedex). Deux oligonucléotides servant d'amorce pour la polymérisation ont été synthétisés.

- DC1:

```
                        start
                       ⌒‿⌒
        5'- t t a g a t c t A T G A T G C T T T T G C A A G C T T T C C T T – 3'
```

- DC2:

```
                    stop              stop
                   ⌒‿⌒              ⌒‿⌒
        5'– t t a g a t c t T T T A T A A C C T C T A T T T T A C T T C C C T – 3'
```

DC1 est complémentaire du brin anti-sens au niveau du codon d'initiation ATG.

DC2 est complémentaire du brin sens et comprend les deux codons STOP de la partie terminale de la région codante du gène *SUC 2*.

Un site de restriction Bgl II et deux nucléotides débordants ont été ajoutés afin de permettre le clonage ultérieur des séquences amplifiées dans le plasmide $pUC_{18}$ [référence Yannish - Perron et al (1985) Gene 33, 103-119].

L'ADN total de départ a été préparé à partir de la souche haploïde HO 37 déposée dans la CNCM sous le numéro I-1076, par des méthodes classiques d'isolement d'ADN de levure ["Methods in Yeast Genetics" - A laboratory Course Manual - Rose, M.D., Winston, F. and Hieter, P. - Cold Spring Harbor Laboratory Press, Cold Spring Harbor, New York (1990)]. Cette souche haploïde contient un seul gène *SUC*: le gène *SUC 2* porté par le chromosome IX. La concentration d'ADN de la solution de travail est de 300 μg/ml.

L'amplification a été réalisée sur 1 μg d'ADN en présence des deux amorces DC1 et DC2 (concentration finale pour chacune = 1 μM) et de 2,5 unités d'Ampli Taq. Le programme d'amplification utilisé a été le suivant:

```
        a) Dénaturation 7 min à 95°C

           Pause = ajout de l'enzyme Ampli Taq (2,5 U)



        b) 1 min à 95°C (dénaturation)             }
           2 min à 55°C (hybridation des amorces)  } 35 cycles
           5 min à 72°C (polymérisation)           }
        c) 7 min à 72°C.
```

1.2 <u>Modifications post-PCR = destinées à obtenir une bonne efficacité de clonage du produit de PCR</u>

- Elimination des sous-produits de la réaction de PCR (nucléotides, enzyme, sels, oligonucléotides) par le kit Gene Clean (BIO 101, distribué par OZYME).
- Remplissage des extrémités par la Klenow Polymerase et phosphorylation des extrémités par la T4 Polynucléotide kinase.
- Nouvelle purification de l'ADN par le kit Gene Clean.
- Ligation du produit de PCR (rendu bouts francs et phosphorylé) sur lui-même par la T4 DNA ligase.
- Digestion du produit de ligation par Bgl II (sites inclus dans les oligonucléotides).
- Ligation du gène *SUC 2* / Bgl II dans le plasmide pUC$_{18}$ ouvert au niveau de son site unique Bam H1 [référence: Yannish-Perron, C., Vieira, J. and Messing, J. (1985) Gene 33, 103-119].

Les enzymes de restriction et de modification sont fournies par Boehringer Mannheim GmbH (Postfach 310120, D-6800 Mannheim 31, Germany) et par New England Biolabs distribué en France par OZYME (Avenue Franklin, 78180 Montigny-le-Bretonneux).

Les techniques utilisées sont décrites dans Maniatis, T., Fristsch, E.F. and Sambrook, J. (1982) Cold Spring Harbor Laboratory, Cold Spring Harbor, New York.

1.3 <u>Disruption du gène *SUC 2* par le gène pTEF1/Tn5 Ble / tCYC1 (PH$^R$) conférant la résistance à la phléomycine:</u>

Introduction du gène de résistance à la phléomycine *Tn5 Ble* sous promoteur et terminateur levure *(pTEF1 et tCYC1)* au niveau du site Bam H1 unique de *SUC 2*.

- Isolement du gène *pTEF1 / Tn5Ble / tCYC1* du plasmide pUT 332 par digestion Kpn I et Hind III.
  Origine du plasmide: fourni par CAYLA, revendeur de la phléomycine (Centre Commercial de Gros, Avenue de Larrieu, 31094 Toulouse).
  Référence: Gatignol, A., Dassain, M. and Tiraby, G. - "Cloning of Saccharomyces cerevisiae promoters using a probe vector based on phleomycin resistance" - Gene (1990) 91, 35-41.
- Remplissage des extrémités du gène *pTEF1 / Tn5 Ble / tCYC1* (pour rendre le gène bouts francs) par la Klenow Polymérase.
- Ouverture du plasmide pUC$_{18}$-*SUC 2* (décrit en 12) par Bam H1 (site unique situé au milieu de *SUC 2*). Remplissage des extrémités Bam H1 par la Klenow Polymérase et déphosphorylation par la CIP (Calf Intestine Phosphatase).
  ==> obtention de bouts francs déphosphorylés.
- Clonage du gène *pTEF1 / Tn5 Ble / tCYC1* bouts francs dans le plasmide pUC$_{18}$ - *SUC 2* au niveau de son site Bam H1 rempli et déphosphorylé.
  ==> obtention du plasmide pUC$_{18}$ - *suc 2::PH$^R$* décrit en annexe 1.

1.4 Préparation de l'ADN transformant

- Digestion du plasmide pUC$_{18}$ - *suc2::PH$^R$* par KpnI et Hind III. (PH$^R$ signifie: résistant à la phléomycine)
- Isolement du fragment Kpn I / Hind III de 2 355 pb par le kit Gene Clean (BIO 101, distribué par OZYME).

1.5 Disruption d'un gène *SUC* dans une souche haploïde, diploïde ou aneuploïde de levure.

Par exemple, la disruption peut être effectuée de la manière suivante:

- Transformation de la souche par électroporation (électropulsateur JOUAN) selon la technique décrite dans Biotechnology (1990) 8, 223-227 - Meilhoc, E., Masson, J.M. and Tessie, J. - "High efficiency transformation of intact yeast cells by electric field pulses".
  Paramètres de transformation:
  10$^8$ cellules / 50 µl - une impulsion de 15 msec - 2500 V/cm - quantité d'ADN transformant: 1 µg.
- Sélection des clones transformants pour la résistance à la phléomycine sur boîte de Pétri contenant un milieu YEG gélosé supplémenté à 100 µg/ml de phléomycine (CAYLA) et une teneur basse en invertase (cf. test colorimétrique au chlorure de triphényl-tétrazolium décrit plus haut dans la présente description).

Bien entendu, d'autres méthodes de transformation comme la technique au chlorure de lithium peuvent être utilisées, également d'autres marqueurs que la phléomycine peuvent être utilisés.

1.6 Construction d'une souche de levure parfaitement homologue ne contenant pas que de l'ADN de levure.

1.6.1 Création d'un gène *suc 2Δ* délété

Par exemple, on peut procéder de la manière suivante:

- Le plasmide pUC$_{18}$ - *SUC 2* décrit en 12 est ouvert au niveau du site unique Bam H1 situé au milieu de la partie codante de *SUC 2* (position nucléotide 787 en comptant le A de l'ATG comme nucléotide n°1).
- Les extrémités Bam H1 générées servent de point de départ pour l'exonucléase Bal 31 qui dégrade de façon récurrente et simultanée les deux brins du duplex DNA dans les deux sens, créant ainsi une délétion dans *SUC 2*. L'hydrolyse par Bal 31 est menée dans les conditions décrites par Maniatis et al. La réaction est arrêtée dès que 150 nucléotides environ ont été éliminés de part et d'autre du site Bam H1.

L'ADN est ensuite réparé par la Klenow polymérase pour générer des bouts francs et mis en ligation pour donner le plasmide pUC$_{18}$ - *suc 2Δ*. Ce plasmide est amplifié en transformant la souche d'E. coli DH5α (commercialisée par GIBCO-BRL, 14 rue des Oziers, BP 7050, 95051 Cergy-Pontoise Cedex). La délétion est contrôlée par cartographie de restriction du plasmide pUC$_{18}$ - *suc 2Δ* obtenu.

Bien entendu, la non expression du gène SUC peut être obtenue également par d'autres moyens techniques tels que l'introduction d'une mutation ponctuelle, par exemple par PCR, conduisant à l'introduction de codons stop au début de la partie codante du gène SUC, ce qui entraîne un arrêt prématuré de la traduction. On peut également intervenir par mutation dirigée sur le promoteur afin que la transcription du gène ne s'effectue pas ou peu.

1.6.2. Construction d'une souche haploïde, diploïde ou aneuploïde à invertase basse par recombinaison génétique homologue ne mettant en jeu que de l'ADN de levure.

La construction d'une telle souche peut être réalisée par une technique classique de cotransformation, par exem-ple:

- Le plasmide pUC$_{18}$ - *suc 2Δ* est digéré par Kpn I et Hind III. Le fragment Kpn I - Hind III de *suc 2Δ* (environ 900 pb) est ensuite purifié par le kit Gene Clean.
- La souche de levure dont on veut abaisser son potentiel d'activité invertase est cotransformée par électroporation par le fragment Kpn I - Hind III de *suc 2Δ* décrit ci-dessus et le plasmide pUT 332 qui sont employés dans un rapport molaire 10:1. Le pUT 332, déjà décrit, est un plasmide navette bactérie-levure contenant le gène de résistance à la phléomycine. La sélection des transformants *PH$^R$* (= résistants à la phléomycine) est obtenue par l'étalement des cellules transformées sur boîtes de Pétri de milieu YEG gélosé supplémenté à 100 µg/ml en phléomycine (CAYLA). Les clones PH$^R$ ayant une activité invertase diminuée sont sélectionnés à l'aide du test colorimétrique au chlorure de triphényl-tétrazolium décrit plus haut.

- Le clone PH[R] possédant l'activité invertase basse recherchée est débarrassé du plasmide pUT 332 par culture pendant un nombre important de générations sur milieu liquide YPG (extrait de levure 1%, bactopeptone 2%, glucose 2%) sans phléomycine. On procède ensuite à la sélection d'un sous-clone devenu sensible à la phléomycine.

L'absence de toute trace d'ADN plasmide bactérien ou de marqueur de résistance peut être vérifiée par une technique classique de Southern blot (Maniatis et al, 1982) à l'aide du plasmide pUT 332 marqué par exemple au $^{32}$P. L'événement d'intégration du gène *suc 2Δ* en lieu et place d'un gène *SUC* intact peut également être contrôlé par la même technique en utilisant le gène *SUC 2* comme sonde.

La technique de cotransformation décrite ci-dessus peut aussi être, par exemple, utilisée pour remplacer le gène suc *2::PH*[R], intégré dans une souche de levure, par le gène *suc 2Δ* préparé comme décrit en 1.6.2. Dans ce cas, le fragment Kpn I - Hind III du plasmide pUC$_{18}$ - *suc 2Δ* est introduit par cotransformation en présence d'un plasmide navette bactérie-levure contenant un gène marqueur de résistance à un antibiotique autre que la phléomycine comme par exemple la généticine, la cycloheximide, un herbicide... Dans ce cas, on recherche parmi les clones transformants résistants à l'antibiotique choisi ceux qui sont devenus sensibles à la phléomycine et on élimine ensuite, comme décrit ci-dessus, le plasmide navette conférant la seconde résistance.

Le remplacement d'un gène *SUC* d'une souche de levure par un gène *SUC* muté ou délété peut également être obtenu par des techniques autres que la technique de cotransformation comme par exemple la technique de "Jettisonning" décrite dans le brevet européen EP 163 491.

**2 - Applications du protocole décrit ci-dessus à la souche 3597.**

Les souches très rapides sur pâtes sans sucre répondant aux critères de sélection définis pour les souches parentes du premier groupe:

- dégagement gazeux d'au moins 170 ml et de préférence d'au moins 180 ml en 2 heures dans le test A$_1$,
- bon rendement sur mélasses,
- assez faible osmosensibilité (ou bonne osmotolérance), évaluée selon les tests décrits ci-dessus,

donnent des ségrégeants (haploïdes) ayant des activités:

- maltose-perméase constitutive, c'est-à-dire maltose-perméase mesurée après croissance sur glucose: au moins 7 unités;
- maltase constitutive, c'est-à-dire maltase mesurée après croissance sur glucose: au moins 80 unités;
- invertase en général plus de 20 unités.

On a croisé une série des haploïdes obtenus correspondant à ces caractéristiques, c'est-à-dire ayant une activité invertase supérieure à 20 unités avec l'haploïde HO 37.

On a obtenu des souches hybrides qui, après culture comme décrit dans les exemples 2 et 3, présentaient des activités élevées dans le test A$_1$ (au moins 180 ml en 2 heures), mais moyennes dans les tests A$_5$ et A$_6$. Une souche, la souche 3597 a paru particulièrement intéressante du fait de son activité dans le test A$_1$ et de son rendement élevé sur mélasses. Il a été vérifié que cette souche 3597 donnait un rapport Test A$_{10}$ / Test A$_9$ d'au moins 0,45 et qu'elle était donc peu osmosensible, mais qu'elle donnait systématiquement des levures ayant une activité invertase d'au moins 50 unités.

Il a été décidé de disrupter au moins un des gènes *SUC* de cette souche. Le travail a été mené comme décrit ci-dessus. Les essais de culture selon les exemples 3 et 4 ont été menés sur la souche 3597 D 78/1 qui contient toujours le gène de résistance à la phléomycine, celui-ci pouvant être éliminé par la méthode décrite ci-dessus. La disruption d'un seul gène *SUC* sur les trois gènes *SUC* portés par cette souche hybride a conduit à diviser son activité invertase par 5 et à augmenter de 20% l'activité de cette souche dans les tests A$_5$ et A$_6$. La souche 3597 D 78/1 donne des levures ayant une activité invertase de l'ordre de 10 unités.

La souche 3597 D 78/1 cultivée selon l'exemple 3 donne une levure fraîche à environ 30% de matières sèches ayant les caractéristiques suivantes:

| Azote/matières sèches | Test A$_1$ | Test A$_5$ | Test A$_6$ |
|---|---|---|---|
| 9,12 | 195 | 149 | 192 |

Cette souche transformée 3597 D 78/1 a été déposée dans la CNCM sous le n° I-1202 le 13 avril 1992 et au NCYC (National Collection of Yeast Cultures - AFRC Institute of Food Research - Norwich Laboratory - Norwich Research Park - Colney Lane - Norwich NR4 7UA - U.K.) sous le n° NCYC 2383 le 15 avril 1992.

Cet exemple de transformation a été fait sur une souche hybride pour démontrer qu'à partir d'une souche diploïde ou aneuploïde de levure de panification qui donne des activités malto-perméase constitutive et maltase constitutive élevées, qui est peu osmosensible (c'est-à-dire qui est osmotolérante) mais qui présente une activité invertase supérieure à 10 unités, on obtient des nouvelles souches selon l'invention en abaissant sa teneur en invertase par biologie moléculaire, plus particulièrement par disruption d'un ou plusieurs de ses gènes codant pour l'invertase (gène SUC). De préférence, cette technique sera appliquée aux haploïdes (ségrégeants), et de manière préférentielle aux haploïdes contenant au maximum 1 ou 2 gènes *SUC*. On devient ainsi certain d'obtenir à partir des souches rapides, qui sont peu osmosensibles, c'est-à-dire qui répondent aux tests décrits, des haploïdes répondant à l'ensemble des critères requis et qui donnent les nouvelles souches de levure de panification correspondant à l'invention.

## ANNEXE 1

Construction du vecteur de disruption *suc 2 :: PH^R*

**EXEMPLE 6**

**Caractérisation des nouvelles levures de panification obtenues. Obligation de recourir à des tests comparatifs.**

Les levures de panification ne peuvent pas être caractérisées autrement que par leur fonction principale: leur activité fermentative, c'est-à-dire leur pouvoir de dégagement gazeux dans des tests conventionnels reproduisant différentes conditions de la fermentation panaire. La signification de ces tests est discutée dans l'exemple 7.

Les levures de panification ne peuvent pas être caractérisées autrement, elles sont en effet obtenues par la mise en oeuvre d'au moins deux moyens:

- une souche,
- un procédé de culture de cette souche, complétés par des procédés de récolte, filtration, extrusion, séchage, conditionnement qui doivent respecter les cellules de levures.

La souche est un moyen essentiel, mais d'une part ce n'est qu'un moyen, les caractéristiques du procédé de culture sont également importantes, et d'autre part, les procédés de construction de nouvelles souches conduisent en général à plusieurs souches équivalentes.

Le procédé de culture se compose de plusieurs phases anaérobies, puis aérobies de multipliation de la souche de départ, comme décrit dans les ouvrages de référence. La phase la plus importante est le dernier cycle de multiplication conduisant à la levure pressée destinée à être commercialisée sous forme de levure pressée à environ 30% de matières sèches ou a été séchée par un séchage ménageant à au moins 94% de matières sèches.

Les paramètres caractéristiques de ce dernier cycle de multiplication, dont la durée est de l'ordre de 14 heures, sont:

- la dilution du milieu de culture en fin de cycle (ou encore la concentration du milieu de culture en cellules et en non-sucres),
- le taux de multiplication horaire moyen pendant la durée de ce dernier cycle de multiplication, étant entendu que le taux horaire de multiplication est plus élevé en début de cycle et abaissé en fin de ce cycle, le choix de la courbe de multiplication permettant de régler le taux de bourgeons, la composition de la levure;
- la composition recherchée pour les levures au niveau de la récolte;

étant entendu qu'avec ces caractéristiques, l'homme de l'art, à la lumière des documents cités dans la présente demande et notamment d'une part les brevets antérieurs de la Demanderesse, et d'autre part les ouvrages de référence dont notamment l'ensemble des ouvrages écrits ou publiés par Gerald Reed, a tous les éléments pour définir expérimentalement, dans le cadre de ses connaissances normales, l'ensemble du schéma de production. La production de levures fraîches très actives, de levures sèches correspondent à des schémas de production différents, les compositions finales recherchées pour la levure récoltée étant assez différentes. Chaque souche demande de légères adaptations. La composition de la mélasse est aussi une variable importante, notamment le levurier doit vérifier l'absence d'éléments toxiques ou inhibiteurs dans les mélasses.

Ces mises au point de schéma, qui relèvent du travail normal de l'homme de l'art, doivent être faites en utilisant des souches témoins bien connues et en mesurant le maximum de paramètres.

A titre d'essais pour essayer d'éliminer l'incidence mélasses, des essais de réalisation du dernier cycle de multiplication ont été réalisés sur milieu synthétique classique avec des levures d'ensemencement produites selon une méthode classique.

Pour un cycle de multiplication conduisant à une coulée de 850 g de sucre en 14 heures, un pied de cuve de 4 kg contenant les sels et vitamines suivants a été réalisé:

| Sulfate de potassium | $K_2SO_4$ | 101 g |
|---|---|---|
| Chlorure de calcium | $CaCl_2$ | 3,4 g |
| Chlorure de magnésium | $MgCl_2$ | 14,5 g |
| Sulfate de sodium | $Na_2SO_4$ | 23 g |
| Ammonium fer sulfate | $(NH_4)_2Fe(SO_4)_2,6H_2O$ | 2590 mg |
| Sulfate d'aluminium et de potassium | $K\,AL(SO_4)_2,12H_2O$ | 149 mg |
| Sulfate de zinc | $ZnSO_4, 7H_2O$ | 650 mg |
| Sulfate de cuivre | $CuSO_4, 5H_2O$ | 1,5 mg |

(suite)

| | | |
|---|---|---|
| Chlorure de cobalt | $CoCl_2$, $6H_2O$ | 2,1 mg |
| Borate de sodium | $Na_2B_4O_7$, $10H_2O$ | 2,2 mg |
| Chlorure de manganèse | $MnCl_2$, $4H_2O$ | 0,92 mg |
| Oxyde de molybdène | $MoO_3$ | 0,026mg |
| Acide nicotinique | | 188 mg |
| Mésoinositol | | 1880 mg |
| Vitamine B1 | | 86 mg |
| Vitamine B2 | | 3,7 mg |
| Vitamine B6 | | 24 mg |
| Biotine | | 1,02mg |
| Acide paraaminobenzoïque | | 11,4 mg |
| Pantothénate de calcium | | 18,8 mg |

On ajoute également à ce milieu 10 g d'extrait de levure en poudre pour milieu de culture de type DIFCO, ainsi que la levure d'ensemencement.

Les 850 g de sucre, l'azote et le $P_2O_5$ nécessaires pour la culture en fed batch sont coulés selon une courbe de multiplication des levures, correspondant aux conditions définies dans l'exemple 3 ou 4 selon le cas. Le pH est régulé par la soude entre 4 et 7. Le volume final de la cuve lors de la récolte est de 7,5 litres contenant environ 1500 g de levure à 30% de matières sèches.

Les résultats suivants ont été obtenus:

| Souche | N/ms | A1 | A5 | A6 | A'1 | A'5 | A'6 |
|---|---|---|---|---|---|---|---|
| | | Levures fraîches | | | Levures sèches | | |
| NCYC 995 | 7,6 | 156 | 100 | 125 | 124 | 70 | 90 |
| NCYC 996 | 7,2 | 105 | 117 | 153 | 90 | 95 | 126 |
| 3493 | 8,3 | 170 | 140 | 199 | 128 | 111 | 166 |
| 3493 | 8,9 | 189 | 150 | 207 | | | |

On remarquera que, dans ces conditions expérimentales, on retrouve le même classement et les mêmes tendances. La reproductibilité des productions biologiques peut être maîtrisée à l'intérieur d'un même laboratoire ou d'un même centre de production, c'est un problème délicat entre laboratoires et centres de production différents. En conséquence, plusieurs essais soigneux sont nécessaires pour optimaliser les conditions. Une ou plusieurs souches témoins, bien connues, doivent toujours être introduites dans les essais, de manière à avoir au moins un ensemble de données en valeurs relatives.

## EXEMPLE 7

**Signification des tests d'activité fermentative**

Les différents tests utilisés pour mesurer l'activité des levures de panification sont discutés dans le chapitre VII 1 B intitulé "Appréciation du pouvoir fermentaire", pages 449 à 464 du Guide Pratique d'Analyses dans les Industries des Céréales, coordonné par B. GODON et W. LOISEL, éditions Techniques et Documentation Lavoisier/Apria 1984. La méthode de Burrows et Harrison y est décrite comme la méthode de choix pour les levureries.

Les tests retenus dans la présente demande de brevet sont:

- les tests $A_1$ et $A'_1$ qui correspondent à la méthode décrite en détail pages 455 à 458 de cet ouvrage, et qui sont la reprise à quelques variantes près de la méthode originale telle que décrite par ses auteurs en 1959. Ils correspondent à une pâte sans sucre;
- les tests $A_5$ et $A'_5$, $A_6$ et $A'_6$ qui correspondent à la mise en oeuvre de pâtes sucrées.

Ces derniers tests ont été préférés aux tests $A_2$, $A'_2$, $A_3$, $A'_3$, $A_4$, $A'_4$ qui sont apparus à l'expérience moins instructifs. Quelques essais ont été faits en reprenant les tests $A_3$, $A'_3$ et $A_4$ et $A'_4$. Leurs résultats sont décrits dans le tableau ci-dessous, dans lequel on rappelle les résultats obtenus dans ces tests avec les souches NCYC 890, NCYC 995 et NCYC 996 à titre de comparaison.

EP 0 511 108 B1

| Souche | N/MS | Levures fraîches 30-35% m.s. | | | | | Levures sèches 95% m.s. | | | | |
|---|---|---|---|---|---|---|---|---|---|---|---|
| | | A1 2 heures | A3 1 heure | A4 1 heure | A5 2 heures | A6 2 heures | A'1 2 heures | A'3 1 heure | A'4 1 heure | A'5 2 heures | A'6 2 heures |
| 3493 | 8,00 | 170 | 70 | | 140 | 198 | 137 | 57 | | 113 | 175 |
| 3493 | 8,06 | 177 | 75 | | 144 | 198 | 139 | 58 | | 115 | 157 |
| 3603 | 7,94 | 182 | 73 | 48 | 147 | 220 | 122 | 51 | 35 | 105 | 150 |
| 3608 | 8,60 | 208 | 82 | 54 | 160 | 233 | 130 | 52 | 33 | 102 | 159 |
| 3608 | 7,90 | 193 | 78 | 50 | 151 | 226 | 135 | 60 | 38 | 115 | 170 |
| NCYC 995 | 7,5 | 154 | | 32 | 100 | 125 | 131 | | 26,3 | 82 | 100 |
| NCYC 996 | 7,2 | 105 | | 41 | 125 | 165 | 91 | | 34 | 100 | 130 |
| NCYC 890 | 7,8 | 145 | 58 | 33 | | | 128 | 50 | 27,5 | | |

Par ailleurs, dans la demande de brevet européen n° 88 201870.8 publiée sous le n° 0 306 107, qui vise par des moyens différents, l'obtention de souches à large spectre, ce sont des tests, spécifiques à la Demanderesse de ce brevet européen, intitulés B, C et B', C' qui sont utilisés. La levure de panification obtenue avec une souche transformée par intégration de vecteurs homologues n'est toujours pas proposée à la vente, selon les déclarations les plus récentes d'un porte-parole de la Demanderesse dudit brevet. Aucune souche transformée n'est déposée dans un centre de collections. Les tests B et C correspondent à des tests sur pâte normale sans sucre, et les tests B' et C' correspondent à des tests sur pâte à 30% de sucre. Faute de pouvoir se procurer des échantillons, un essai de corrélation entre les résultats donnés:

- par lesdits tests B et C d'une part, et les tests $A_1$ et $A'_1$ d'autre part,
- par lesdits tests B' et C' d'une part, et les tests $A_6$ et $A'_6$ d'autre part,

a été fait. Compte tenu des résultats de cet essai de corrélation, on peut établir le tableau suivant:

|  | Levure fraîche comprimée à environ 30% de matières sèches | | | |
|---|---|---|---|---|
|  | B | A1 | B' | A6 |
| Souche A de la demande 306 107 | 311 | 155 | 190 | 122 |
| Souche ApGb p2RBRRO1=1 | 367 | 184 | 190 | 122 |
| Souche 3493 | 396 | 198 | 333 | 215 |
| Souche 3608 | 420 | 210 | 364 | 235 |

On notera que très peu de valeurs absolues sont données et qu'aucun essai de séchage n'est décrit dans la demande de brevet européen 306 107. Quoiqu'il en soit, il apparaît que la nouvelle levure fraîche comprimée, objet de la présente invention, par rapport à la levure fraîche comprimée obtenue avec la souche ApGb-p2RBRR01=01:

- est au moins aussi active sur pâte normale,
- est beaucoup plus active sur pâte sucrée.

Ces conclusions peuvent être extrapolées aux levures sèches. Ces conclusions sont normales, si on prend en compte que les meilleures levures sur pâtes très sucrées ont une activité sur pâte sans sucre inférieure à la moitié de celles des meilleures levures spécialisées pour pâte sans sucre. Le gain maximum sur pâtes sans sucre espéré par la méthode de transformation employée dans la demande de brevet européen publiée sous le n° 306 107 est de quelques dizaines de pour cent. Cette méthode de transformation ne peut conduire sur la base des résultats décrits qu'à des levures ayant des performances inférieures à celles de la présente invention.

**Revendications**

1. Nouvelles souches de levure de panification à large spectre ayant

- un bon rendement de multiplication,
- une bonne assimilation de l'azote,
- une bonne activité fermentative du glucose,
- de préférence une bonne résistance au séchage,

caractérisées par le fait qu'elles présentent simultanément les activités enzymatiques suivantes:

- activité maltose-perméase après croissance de la levure sur milieu glucose, en l'absence de maltose (Test $T_1$): au moins 9 unités,
- activité maltase après croissance de la levure sur milieu glucose, en l'absence de maltose (Test $T_2$): au moins 80 unités et, de préférence, au moins 90 unités,
- activité invertase (Test $T_3$): moins de 10 unités et, de préférence, plus de 2 unités.

**2.** Nouvelles souches de levure de panification à large spectre selon la revendication 1, caractérisées par le fait qu'elles présentent simultanément les activités enzymatiques suivantes:

- activité maltose-perméase après croissance de la levure sur milieu maltose (Test T'$_1$), au moins 12 unités,
- activité maltase après croissance de la levure sur milieu maltose (Test T'$_2$), au moins 200 unités.

**3.** Procédé de construction de nouvelles souches de levure de panification à large spectre selon l'une des revendications 1 et 2, caractérisé par le fait que

a) que l'on sélectionne les souches parentes parmi celles:

- qui, de préférence, appartiennent au groupe des souches osmotolérantes actives sur pâtes sucrées qui sont susceptibles de donner des levures fraîches ayant un dégagement gazeux d'au moins 160 ml en 2 heures dans le test A$_6$, ou qui appartiennent au groupe des souches actives sur pâte normale qui, d'une part, sont susceptibles de donner des levures fraîches ayant un dégagement gazeux d'au moins 170 ml et de préférence d'au moins 180 ml en 2 heures dans le test A$_1$ et qui, d'autre part, sont peu osmosensibles, c'est-à-dire qui permettent de produire des levures fraîches:

  . soit qui donnent au moins 70 ml et de préférence au moins 80 ml et plus préférentiellement au moins 90 ml en 2 heures dans un test A$_5$ où le saccharose a été remplacé par une quantité égale de glucose,
  . soit qui donnent un rapport Test A$_{10}$/Test A$_9$ au moins égal à 0,40 et de préférence au moins égal à 0,45,

- qui donnent un rendement acceptable sur mélasses y compris à des taux de multiplication horaire élevés de l'ordre de 1,20,
- qui assimilent aisément l'azote et sont susceptibles de donner des levures de panification dans des conditions courantes de culture avec des teneurs en azote sur matières sèches de l'ordre de 9%, c'est-à-dire entre 8,6% et 9,2% et
- qui, de préférence, sont de plus stables au séchage,

b) que l'on fait sporuler les souches ainsi sélectionnées,
c) que l'on sélectionne parmi les haploïdes (ségrégeants) ainsi obtenus, ceux qui auront de plus un potentiel élevé pour avoir des propriétés maximales sur les types de pâtes boulangères pour lesquels la souche parente n'est pas performante, c'est-à-dire les haploïdes qui simultanément :

- se colorent en bleu dans le test de coloration bleue réalisé sur boîte de Pétri lorsqu'ils sont cultivés sur un milieu à base de glucose comme le milieu YEG (Yeast Extract 0,5%, glucose 2%, agar 3%) en présence de 5-bromo-4-chloro-3-indolyl-alpha-D-gluco-pyrano-side ou X alpha-glu,
- présentent un non développement d'une coloration rose dans le test pour déterminer le taux d'invertase d'après Trumbly,
- ont un taux d'invertase (Test T$_3$) inférieur à 10 unités et, de préférence, supérieur à 2 unités,

et qui présentent les activités enzymatiques suivantes:

- maltose-perméase après culture sur glucose (Test T$_1$): au moins 3 unités et, de préférence, au moins 7 unités,
- maltase après culture sur glucose (Test T$_2$): au moins 40 unités et, de préférence, au moins 80 unités,

d) que l'on croise ensemble les haploïdes ainsi sélectionnés,
e) que l'on sélectionne, parmi les hybrides issus desdits croisements, les souches conformes à la revendication 1 en ayant recours aux tests suivants:

- développement dans le test de coloration bleue réalisé sur boîte de Pétri d'une culture bleue après étalement sur boîte de Pétri YEG en présence de X alpha-glu,
- non développement d'une coloration rose dans le test pour déterminer le taux d'invertase d'après Trumbly,
- maltose-perméase après culture de la levure sur milieu glucose, en l'absence de maltose (Test T$_1$), au moins 9 unités,
- maltose-perméase après culture sur milieu maltose (Test T'$_1$), au moins 12 unités,

- maltase sur milieu glucose, en l'absence de maltose (Test $T_2$), au moins 50 unités, de préférence au moins 80 unités et, encore de préférence, au moins 90 unités,
- maltase sur milieu maltose (Test $T'_2$), au moins 200 unités,
- invertase (Test $T_3$): moins de 10 unités, de préférence plus de 2 unités,

et qu'on opère une sélection supplémentaire parmi les hybrides ainsi sélectionnés par des méthodes conventionnelles, c'est-à-dire par des tests de culture:

- en fonction du rendement,
- en fonction de l'assimilation d'azote,
- en fonction des activités dans les tests $A_1$ et $A_6$,
- en fonction de la résistance au séchage.

4. Procédé de construction de nouvelles souches selon la revendication 3, caractérisé par le fait que les haploïdes (ségrégeants) issus de souches présentant sur pâtes normales des propriétés particulièrement remarquables et peu osmosensibles ont leur niveau d'activité invertase abaissé par les moyens de la biologie moléculaire.

5. Nouvelle levure fraîche de panification susceptible d'être obtenue à l'aide des nouvelles souches de levure de panification selon les revendications 1 ou 2, caractérisée par le fait que:

- dans le test $A_1$ sur 2 heures, elle donne un dégagement gazeux compris entre 170 et 230 ml, de préférence entre 190 et 230 ml,
- dans le test $A_5$ sur 2 heures, elle donne un dégagement gazeux compris entre 130 et 180 ml, de préférence entre 140 et 180 ml et, plus préférentiellement encore, entre 150 et 180 ml,
- dans le test $A_6$ sur 2 heures, elle donne un dégagement gazeux compris entre 170 et 230 ml, de préférence entre 180 et 250 ml et, plus préférentiellement encore, entre 200 et 250 ml.

6. Nouvelle levure sèche de panification susceptible d'être obtenue à l'aide des nouvelles souches de levure de panification selon les revendications 1 ou 2, caractérisée par le fait que:

- dans le test $A'_1$ sur 2 heures, elle donne un dégagement gazeux compris entre 120 et 145 ml, de préférence entre 130 et 145 ml et, plus préférentiellement encore, entre 135 et 145 ml,
- dans le test $A'_5$ sur 2 heures, elle donne un dégagement gazeux compris entre 95 et 130 ml, de préférence entre 100 et 130 ml et, plus préférentiellement encore, entre 110 et 130 ml, et
- dans le test $A'_6$ sur 2 heures, elle donne un dégagement gazeux compris entre 135 et 190 ml, de préférence entre 140 et 190 ml et, plus préférentiellement encore, entre 150 et 190 ml.

7. Haploïde déposé dans la Collection Nationale de Cultures de Microorganismes (CNCM) à l'Institut Pasteur sous le n° I-1076.

8. Haploïde déposé dans la Collection Nationale de Cultures de Microorganismes (CNCM) à l'Institut Pasteur sous le n° I-1075.

9. Haploïde déposé dans la Collection Nationale de Cultures de Microorganismes (CNCM) à l'Institut Pasteur sous le n° I-1074.

10. Procédé selon l'une des revendications 3 et 4, caractérisé par le fait que si des haploïdes issus des souches qui sont susceptibles de donner des levures fraîches peu osmosensibles donnant au moins 170 ml et de préférence au moins 180 ml en 2 heures dans le test $A_1$:

- d'une part, dans le test de coloration bleue réalisé en boîte de Pétri, se colorent en bleu dans le test avec le colorant X alpha glu et ont une activité malto-perméase après culture sur glucose (Test $T_1$) d'au moins 7 unités et une activité maltase après culture sur glucose (Test $T_2$) d'au moins 80 unités,
- d'autre part, présentent une teneur en invertase (Test $T_3$) supérieure à 10 unités,

ladite activité invertase est abaissée par disruption d'au moins un gène SUC desdits haploïdes.

11. Procédé selon l'une des revendications 3, 4 et 10, caractérisé par le fait que l'on sélectionne les haploïdes ayant en plus simultanément les activités enzymatiques suivantes:

- maltose-perméase après culture sur maltose (Test $T'_1$): au moins 12 unités,
- maltase après culture sur maltose (Test $T'_2$): au moins 200 unités.

12. Procédé selon l'une des revendications 4, 10 et 11, caractérisé par le fait que l'on sélectionne parmi les hybrides obtenus, ceux:

- qui donnent lieu, dans le test de coloration bleue réalisé sur boîte de Péri, au développement d'une culture bleue après étalement sur boîte de Pétri YEG en présence de X alpha-glu,
- qui ne donnent pas lieu au développement d'une coloration rose dans le test pour déterminer le taux d'invertase d'après Trumbly,

et qui présentent les activités enzymatiques suivantes:

- maltose-perméase après culture sur milieu glucose, en l'absence de maltose (Test $T_1$), au moins 9 unités,
- maltose-perméase après culture sur milieu maltose (Test $T'_1$), au moins 12 unités,
- maltase sur milieu glucose, en l'absence de maltose (Test $T_2$), au moins 50 unités, de préférence au moins 80 unités et, encore de préférence, au moins 90 unités,
- maltase sur milieu maltose (Test $T'_2$), au moins 200 unités,
- invertase (Test $T_3$): moins de 10 unités, de préférence plus de 2 unités,

étant entendu que, parmi les hybrides ainsi sélectionnés, on opère une sélection supplémentaire par des méthodes usuelles:

- en fonction du rendement,
- en fonction de l'assimilation d'azote,
- en fonction des activités dans les tests $A_1$ et $A_6$,
- en fonction de la résistance au séchage.

13. Procédé de construction de nouvelles souches de levures de panification à large spectre selon l'une des revendications 3 et 12, caractérisé par le fait que, si un hybride obtenu satisfait à tous les tests de sélection selon la revendication 12, sauf celui concernant l'activité invertase, cette activité invertase est abaissée par disruption d'au moins un des gènes SUC dudit hybride.

14. Procédé de construction de nouvelles souches de levure de panification à large spectre ayant

- un bon rendement de multiplication,
- une bonne assimilation de l'azote,
- une bonne activité fermentative du glucose,
- de préférence une bonne résistance au séchage,

et présentant simultanément les activités enzymatiques suivantes:

- activité maltose-perméase après croissance de la levure sur milieu glucose, en l'absence de maltose (Test $T_1$): au moins 9 unités,
- activité maltase après croissance de la levure sur milieu glucose, en l'absence de maltose (Test $T_2$): au moins 80 unités et, de préférence, au moins 90 unités,
- activité invertase (Test $T_3$): moins de 10 unités et, de préférence, plus de 2 unités,

à partir d'une souche ayant

- un bon rendement de multiplication,
- une bonne assimilation de l'azote,
- une bonne activité fermentative du glucose,
- de préférence une bonne résistance au séchage,

et présentant simultanément les activités enzymatiques suivantes:

- activité maltose-perméase après croissance de la levure sur milieu glucose, en l'absence de maltose (Test $T_1$): au moins 9 unités,
- activité maltase après croissance de la levure sur milieu glucose, en l'absence de maltose (Test $T_2$): au moins 80 unités et, de préférence, au moins 90 unités,
- activité invertase (Test $T_3$): supérieure à 10 unités,

caractérisé par le fait que l'on abaisse son activité invertase à une valeur inférieure à 10 unités et, de préférence, supérieure à 2 unités, en procédant à la disruption d'un ou plusieurs de ses gènes codants pour l'invertase (gènes SUC).

15. Procédé de construction de nouvelles souches de levure de panification à large spectre selon l'une des revendications 1 et 2, caractérisé par le fait que l'on croise ensemble au moins deux des haploïdes selon les revendications 7 à 9 et que l'on sélectionne, parmi les hybrides issus de ces croisements, ceux

- qui donnent lieu au développement, dans le test de coloration bleue réalisé sur boîte de Pétri, d'une culture bleue après étalement sur boîte de Pétri YEG en présence de X alpha-glu,
- qui ne donnent pas lieu au développement d'une coloration rose dans le test pour déterminer le taux d'invertase d'après Trumbly,

et qui présentent les activités enzymatiques suivantes:

- maltose-perméase après culture sur milieu glucose, en l'absence de maltose (Test $T_1$), au moins 9 unités,
- maltose-perméase après culture sur milieu maltose (Test $T'_1$), au moins 12 unités,
- maltase sur milieu glucose, en l'absence de maltose (Test $T_2$), au moins 50 unités, de préférence au moins 80 unités et, encore de préférence, au moins 90 unités,
- maltase sur milieu maltose (Test $T'_2$), au moins 200 unités,
- invertase (Test $T_3$): moins de 10 unités, de préférence plus de 2 unités,

étant entendu que, parmi les hybrides ainsi sélectionnés, on opère une sélection supplémentaire par des méthodes usuelles:

- en fonction du rendement,
- en fonction de l'assimilation d'azote,
- en fonction des activités dans les tests $A_1$ et $A_6$,
- en fonction de la résistance au séchage.

16. Nouvelle souche de levure de panification déposée dans la Collection Nationale de Cultures de Microorganismes à l'Institut Pasteur sous le n° I-1073.

17. Nouvelle souche de levure de panification déposée dans la Collection Nationale de Cultures de Microorganismes à l'Institut Pasteur sous le n° I-1072.

18. Nouvelle souche de levure de panification déposée dans la Collection Nationale de Cultures de Microorganismes à l'Institut Pasteur sous le n° I-1071.

19. Nouvelle souche de levure déposée dans la Collection Nationale de Cultures de Microorganismes à l'Institut Pasteur sous le n° I-1202 et à la National Collection of Yeasts Cultures sous le n° NCYC 2383.

20. Nouvelle souche de levure de panification obtenue selon le procédé de la revendication 14.

**Patentansprüche**

1. Neue Backhefestämme mit breitem Spektrum, welche

- eine gute Vermehrung,

- eine gute Stickstoffaufnahme,

- eine gute Glucose-fermentierende Aktivität,

- bevorzugt eine gute Trockenbeständigkeit

haben, dadurch gekennzeichnet, daß sie gleichzeitig die nachfolgenden enzymatischen Aktivitäten aufweisen:

- Maltosepermease-Aktivität nach Wachstum der Hefe auf Glucosemedium in Abwesenheit von Maltose (Test $T_1$): mindestens 9 Units,

- Maltase-Aktivität nach Wachstum der Hefe auf Glucosemedium in Abwesenheit von Maltose (Test $T_2$): mindestens 80 Units und bevorzugt mindestens 90 Units,

- Invertase-Aktivität (Test $T_3$): weniger als 10 Units und bevorzugt mehr als 2 Units.

2. Neue Backhefestämme mit breitem Spektrum nach Anspruch 1, dadurch gekennzeichnet, daß sie gleichzeitig die nachfolgenden enzymatischen Aktivitäten aufweisen:

- Maltosepermease-Aktivität nach Wachstum der Hefe auf Maltosemedium (Test $T'_1$), mindestens 12 Units,

- Maltase-Aktivität nach Wachstum der Hefe auf Maltosemedium (Test $T'_2$), mindestens 200 Units.

3. Verfahren zur Herstellung neuer Backhefestämme mit breitem Spektrum nach einem der Ansprüche 1 und 2, dadurch gekennzeichnet, daß

   a) man die Mutterstämme auswählt unter denjenigen,

   - die bevorzugt der Gruppe der osmotoleranten, auf zuckerhaltigen Teigen aktiven Stämme angehören, welche dazu geeignet sind, Frischhefen mit einer Gasentwicklung von mindestens 160 ml in 2 Stunden in Test $A_6$ zu ergeben, oder die der Gruppe der auf normalen Teigen aktiven Stämme angehören, welche einerseits dazu geeignet sind, Frischhefen mit einer Gasentwicklung von mindestens 170 ml und bevorzugt mindestens 180 ml in 2 Stunden in Test $A_1$ zu ergeben, und welche andererseits gering osmosensibel sind, d.h. welche es gestatten Frischhefen zu erzeugen,

     . die entweder mindestens 70 ml und bevorzugt mindestens 80 ml und stärker bevorzugt mindestens 90 ml in einem Test $A_5$, wobei die Saccharose durch eine gleiche Menge Glucose ersetzt worden ist, entwickeln, oder

     . die ein Verhältnis von Test $A_{10}$/Test $A_9$ von mindestens gleich 0,40 und bevorzugt mindestens gleich 0,45 ergeben,

   - die eine akzeptable Ausbeute auf Melassen ergeben, einschließlich stündlicher Vermehrungsraten einer Größenordnung von mehr als 1,20,

   - die leicht Stickstoff aufnehmen und geeignet sind unter üblichen Kulturbedingungen Backhefen mit Stickstoffgehalten in der Trockenmasse in einer Größenordnung von 9 %, d.h. zwischen 8,6 % und 9,2 % zu ergeben, und

   - die bevorzugt weiterhin trockenstabil sind,

   b) man die derart ausgewählten Stämme zum Sporulieren bringt,

   c) man aus den derart erhaltenen Haploiden (Segreganten) diejenigen auswählt, die weiterhin ein erhöhtes Potential aufweisen für maximale Eigenschaften auf den Arten von Backteigen, für die der Mutterstamm nicht leistungsfähig ist, d.h. diejenigen Haploiden, die gleichzeitig:

   - sich in dem in einer Petrischale durchgeführten Blaufärbungstest, wenn sie auf einem Medium auf Glucosebasis wie dem Medium YEG (Hefeextrakt 0,5%, Glucose 2%, Agar 3%) in Gegenwart von 5-Brom-4-chlor-3-indolyl-alpha-D-glucopyranosid oder X-alpha-Glu kultiviert werden, blau färben,

- keine Ausbildung einer rosafarbenen Färbung im Test zur Bestimmung des Invertasegehalts nach Trumbly zeigen,

- einen Invertasegehalt (Test $T_3$) von weniger als 10 Units und bevorzugt von mehr als 2 Units aufweisen,

und die die nachfolgenden enzymatischen Aktivitäten aufweisen:

- Maltosepermease nach Kultur auf Glucose (Test $T_1$): mindestens 3 Units und bevorzugt mindestens 7 Units,

- Maltase nach Kultur auf Glucose (Test $T_2$): mindestens 40 Units und bevorzugt mindestens 80 Units,

d) man die derart ausgewählten Haploiden miteinander kreuzt,

e) man unter den aus diesen Kreuzungen hervorgegangenen Hybriden die Stämme gemäß Anspruch 1 auswählt unter Verwendung der nachstehenden Tests:

- Ausbildung einer blauen Kultur in dem in einer Petrischale durchgeführten Blaufärbungstest nach Plattierung in einer Petrischale auf YEG in Gegenwart von X-alpha-Glu,

- keine Ausbildung einer rosafarbenen Färbung im Test zur Bestimmung des Invertasegehalts nach Trumbly,

- Maltosepermease nach Kultur der Hefe auf Glucosemedium in Abwesenheit von Maltose (Test $T_1$) mindestens 9 Units,

- Maltosepermease nach Kultur auf Maltosemedium (Test $T'_1$) mindestens 12 Units,

- Maltase auf Glucosemedium in Abwesenheit von Maltose (Test $T_2$) mindestens 50 Units, bevorzugt mindestens 80 Units und noch stärker bevorzugt mindestens 90 Units,

- Maltase auf Maltosemedium (Test $T'_2$) mindestens 200 Units,

- Invertase (Test $T_3$): weniger als 10 Units, bevorzugt mehr als 2 Units,

und daß man unter den derart ausgewählten Hybriden mittels herkömmlicher Methoden, d.h. Kulturtests, eine zusätzliche Auswahl durchführt:

- in bezug auf Ausbeute,

- in bezug auf Stickstoffaufnahme,

- in bezug auf Aktivitäten in den Tests $A_1$ und $A_6$,

- in bezug auf Trockenbeständigkeit.

4. Verfahren zur Herstellung neuer Stämme nach Anspruch 3, dadurch gekennzeichnet, daß die Haploiden (Segreganten), welche aus Stämmen hervorgegangenen sind, die auf normalen Teigen besonders herausragende Eigenschaften zeigen und gering osmosensibel sind, einen durch molekularbiologische Mittel erniedrigten Invertase-Aktivitätsgrad haben.

5. Neue Frischbackhefe, welche mit Hilfe der neuen Backhefestämme nach den Ansprüchen 1 oder 2 erhalten werden kann, dadurch gekennzeichnet, daß

- sie in Test $A_1$ innerhalb von 2 Stunden eine Gasentwicklung zwischen 170 und 230 ml, bevorzugt zwischen 190 und 230 ml ergibt,

- sie in Test $A_5$ innerhalb von 2 Stunden eine Gasentwicklung zwischen 130 und 180 ml, bevorzugt zwischen 140 und 180 ml, und noch stärker bevorzugt zwischen 150 und 180 ml ergibt,

- sie in Test $A_6$ innerhalb von 2 Stunden eine Gasentwicklung zwischen 170 und 230 ml, bevorzugt zwischen 180 und 250 ml, und noch stärker bevorzugt zwischen 200 und 250 ml ergibt.

6. Neue Trockenbackhefe, mit Hilfe der neuen Backhefestämme nach den Ansprüchen 1 oder 2 erhalten werden kann, dadurch gekennzeichnet, daß

- sie in Test $A'_1$ innerhalb von 2 Stunden eine Gasentwicklung zwischen 120 und 145 ml, bevorzugt zwischen 130 und 145 ml und noch stärker bevorzugt zwischen 135 und 145 ml ergibt,

- sie in Test $A'_5$ innerhalb von 2 Stunden eine Gasentwicklung zwischen 95 und 130 ml, bevorzugt zwischen 100 und 130 ml, und noch stärker bevorzugt zwischen 110 und 130 ml ergibt,

- sie in Test $A'_6$ innerhalb von 2 Stunden eine Gasentwicklung zwischen 135 und 190 ml, bevorzugt zwischen 140 und 190 ml, und noch stärker bevorzugt zwischen 150 und 190 ml ergibt.

7. Haploide, hinterlegt in der Collection Nationale de Cultures de Microorganismes (CNCM) beim Institut Pasteur unter der Nummer I-1076.

8. Haploide, hinterlegt in der Collection Nationale de Cultures de Microorganismes (CNCM) beim Institut Pasteur unter der Nummer I-1075.

9. Haploide, hinterlegt in der Collection Nationale de Cultures de Microorganismes (CNCM) beim Institut Pasteur unter der Nummer I-1074.

10. Verfahren nach einem der Ansprüche 3 und 4, dadurch gekennzeichnet, daß wenn die Haploiden aus Stämmen hervorgegangen sind, welche geeignet sind gering osmosensible Frischhefen, die innerhalb von 2 Stunden in Test $A_1$ mindestens 170 ml und bevorzugt mindestens 180 ml ergeben:

- einerseits, sich in dem in einer Petrischale durchgeführten Blaufärbungstest im Test mit dem Farbstoff X-alpha-Glu blau färben und eine Maltosepermease-Aktivität nach Kultur auf Glucose (Test $T_1$) von mindestens 7 Units und eine Maltase-Aktivität nach Kultur auf Glucose (Test $T_2$) von mindestens 80 Units aufweisen,

- andererseits einen Invertasegehalt (Test $T_3$) von mehr als 10 Units aufweisen,

die Invertase-Aktivität durch Zerstörung mindestens eines SUC-Gens der Haploiden erniedrigt wird.

11. Verfahren nach einem der Ansprüche 3, 4 und 10, dadurch gekennzeichnet, daß man diejenigen Haploiden auswählt, welche weiterhin gleichzeitig die nachfolgenden enzymatischen Aktivitäten aufweisen:

- Maltosepermease nach Kultur auf Maltose (Test $T'_1$): mindestens 12 Units,

- Maltase nach Kultur auf Maltose (Test $T'_2$): mindestens 200 Units.

12. Verfahren nach einem der Ansprüche 4, 10 und 11, dadurch gekennzeichnet, daß man aus den erhaltenen Hybriden diejenigen auswählt:

- welche in dem in einer Petrischale durchgeführten Blaufärbungstest nach Plattierung in einer Petrischale auf YEG in Gegenwart von X-alpha-Glu die Ausbildung einer blauen Kultur zeigen,

- welche im Test zur Bestimmung des Invertasegehalts nach Trumbly keine Ausbildung einer rosafarbenen Färbung zeigen,

und welche die nachfolgenden enzymatischen Aktivitäten aufweisen:

- Maltosepermease nach Kultur auf Glucosemedium, in Abwesenheit von Maltose (Test $T_1$), mindestens 9 Units,

- Maltosepermease nach Kultur auf Maltosemedium (Test $T'_1$) mindestens 12 Units,

- Maltase auf Glucosemedium, in Abwesenheit von Maltose (Test $T_2$), mindestens 50 Units, bevorzugt mindestens 80 Units und noch stärker bevorzugt mindestens 90 Units,

- Maltase auf Maltosemedium (Test $T'_2$), mindestens 200 Units,

- Invertase (Test $T_3$): weniger als 10 Units, bevorzugt mehr als 2 Units,

wobei verstanden wird, daß man unter den derart ausgewählten Hybriden mittels üblicher Methoden eine zusätzliche Auswahl durchführt:

- in bezug auf Ausbeute,

- in bezug auf Stickstoffaufnahme,

- in bezug auf Aktivitäten in den Tests $A_1$ und $A_6$,

- in bezug auf Trockenbeständigkeit.

13. Verfahren zur Herstellung neuer Backhefestämme mit breitem Spektrum nach einem der Ansprüche 3 und 12, dadurch gekennzeichnet, daß wenn eine erhaltene Hybride allen Auswahltests nach Anspruch 12 genügt, mit Ausnahme des die Invertase-Aktivität betreffenden, diese Invertase-Aktivität durch Zerstörung mindestens eines der SUC-Gene der Hybride erniedrigt wird.

14. Verfahren zur Herstellung neuer Backhefestämme mit breitem Spektrum, welche

- eine gute Vermehrung,

- eine gute Stickstoffaufnahme,

- eine gute Glucose-fermentierende Aktivität,

- bevorzugt eine gute Trockenbeständigkeit

haben und gleichzeitig die nachfolgenden enzymatischen Aktivitäten aufweisen:

- Maltosepermease-Aktivität nach Wachstum der Hefe auf Glucosemedium in Abwesenheit von Maltose (Test $T_1$): mindestens 9 Units,

- Maltase-Aktivität nach Wachstum der Hefe auf Glucosemedium in Abwesenheit von Maltose (Test $T_2$): mindestens 80 Units und bevorzugt mindestens 90 Units,

- Invertase-Aktivität (Test $T_3$): weniger als 10 Units und bevorzugt mehr als 2 Units,

ausgehend von einem Stamm, welcher

- eine gute Vermehrung,

- eine gute Stickstoffaufnahme,

- eine gute Glucose-fermentierende Aktivität,

- bevorzugt eine gute Trockenbeständigkeit

hat und gleichzeitig die nachfolgenden enzymatischen Aktivitäten aufweist:

- Maltosepermease-Aktivität nach Wachstum der Hefe auf Glucosemedium in Abwesenheit von Maltose (Test

$T_1$): mindestens 9 Units,

- Maltase-Aktivität nach Wachstum der Hefe auf Glucosemedium in Abwesenheit von Maltose (Test $T_2$): mindestens 80 Units und bevorzugt mindestens 90 Units,

- Invertase-Aktivität (Test $T_3$): mehr als 10 Units,

dadurch gekennzeichnet, daß man dessen Invertase-Aktivität auf einen Wert von weniger als 10 Units und bevorzugt mehr als 2 Units erniedrigt durch Zerstörung eines oder mehrerer seiner Gene, welche für die Invertase kodieren (SUC-Gene).

15. Verfahren zur Herstellung neuer Backhefestämme mit breitem Spektrum nach einem der Ansprüche 1 und 2, dadurch gekennzeichnet, daß man mindestens zwei Haploiden nach den Ansprüchen 7 bis 9 miteinander kreuzt, und daß man aus den aus diesen Kreuzungen hervorgegangenen Hybriden diejenigen auswählt,

- welche in dem in einer Petrischale durchgeführten Blaufärbungstest nach Plattierung in einer Petrischale auf YEG in Gegenwart von X-alpha-Glu die Ausbildung einer blauen Kultur zeigen,

- welche im Test zur Bestimmung des Invertasegehalts nach Trumbly keine Ausbildung einer rosafarbenen Färbung zeigen,

und welche die nachfolgenden enzymatischen Aktivitäten aufweisen:

- Maltosepermease nach Kultur auf Glucosemedium, in Abwesenheit von Maltose (Test $T_1$), mindestens 9 Units,

- Maltosepermease nach Kultur auf Maltosemedium (Test $T'_1$) mindestens 12 Units,

- Maltase auf Glucosemedium, in Abwesenheit von Maltose (Test $T_2$), mindestens 50 Units, bevorzugt mindestens 80 Units und noch stärker bevorzugt mindestens 90 Units,

- Maltase auf Maltosemedium (Test $T'_2$), mindestens 200 Units,

- Invertase (Test $T_3$): weniger als 10 Units, bevorzugt mehr als 2 Units,

wobei verstanden wird, daß man unter den derart ausgewählten Hybriden mittels üblicher Methoden eine zusätzliche Auswahl durchführt:

- in bezug auf Ausbeute,

- in bezug auf Stickstoffaufnahme,

- in bezug auf Aktivitäten in den Tests $A_1$ und $A_6$,

- in bezug auf Trockenbeständigkeit.

16. Neuer Backhefestamm, hinterlegt in der Collection Nationale de Cultures de Microorganismes beim Institut Pasteur unter der Nummer I-1073.

17. Neuer Backhefestamm, hinterlegt in der Collection Nationale de Cultures de Microorganismes beim Institut Pasteur unter der Nummer I-1072.

18. Neuer Backhefestamm, hinterlegt in der Collection Nationale de Cultures de Microorganismes beim Institut Pasteur unter der Nummer I-1071.

19. Neuer Hefestamm, hinterlegt in der Collection Nationale de Cultures de Microorganismes beim Institut Pasteur unter der Nummer I-1202, und bei der National Collection of Yeast Cultures unter der Nummer NCYC 2383.

20. Neuer Backhefestamm, erhältlich durch das Verfahren nach Anspruch 14.

**Claims**

1. New broad spectrum strains of bread-making yeast having

   a high multiplication yield,
   a good nitrogen assimilation,
   a good glucose fermentation activity
   preferably a good resistance to drying,

   characterized by the fact that they simultaneously have the following enzymatic activities:

   - maltose-permease activity after growth of the yeast on glucose medium, in the absence of maltose (Test $T_1$): at least 9 units,
   - maltase activity after growth of the yeast on glucose medium, in the absence of maltose (Test $T_2$): at least 80 units and, preferably, at least 90 units,
   - invertase activity (Test $T_3$): less than 10 units and, preferably, more than 2 units.

2. New broad spectrum strains of bread-making yeast according to claim 1, characterized by the fact that they simultaneously have the following enzymatic activities:

   - maltose-permease activity after growth of the yeast on maltose medium (Test $T'_1$): at least 12 units,
   - maltase activity after growth of the yeast on maltose medium (Test $T'_2$): at least 200 units.

3. Process for the construction of new broad spectrum strains of bread-making yeast according to one of claims 1 and 2, characterized by the fact

   a) that parent strains are selected from those

   - which belong preferably to the group of osmotolerant strains active on sugared doughs and capable of giving fresh yeasts releasing at least 160 ml of gas in 2 hours in Test $A_6$, or which belong to the group of strains active on normal doughs which, on the one hand, are capable of giving fresh yeasts releasing at least 170 ml and preferably at least 180 ml of gas in 2 hours in Test $A_1$ and which, on the other hand, have low osmosensitivity, i.e. enable production of fresh yeasts which:

     . either give rise to at least 70 ml and preferably at least 80 ml and more preferably at least 90 ml in 2 hours in Test $A_5$ in which the saccharose has been replaced by an equal quantity of glucose,
     . or give rise to a ratio of Test $A_{10}$/Test $A_9$ at least equal to 0.40 and preferably at least equal to 0.45,

   - which provide an acceptable yield on molasses including high hourly multiplication rates of the order of 1.20,
   - which easily assimilate nitrogen and are capable of giving bread-making yeasts under current culture conditions with nitrogen contents of the order of 9%, i.e. between 8.6% to 9.2%, based on the solids content and
   - which preferably are also resistant to drying,

   b) that the parent strains thus selected are sporulated,
   c) that, among the haploids (segregates) thus obtained, the haploids having also a high potential for maximum properties on the type of baking doughs for which the parent strain does not perform, are selected, i.e. the haploids which simultaneously:

   - are coloured blue in the blue coloration test carried out on a Petri dish when they are cultivated on a medium based on glucose such as the YEG medium (Yeast Extract 0.5%, glucose 2%, agar 3%) in the presence of 5-bromo-4-chloro-3-indolyl-alpha-D-gluco-pyranoside or X alpha-glu,
   - show lack of development of a pink color in the test for determining invertase level according to Trumbly,
   - have an invertase level (Test $T_3$) less than 10 units and preferably higher than 2 units,

   and which have the following enzymatic activities:

- maltose-permease activity after culture on glucose (Test $T_1$): at least 3 units, and preferably at least 7 units,
- maltase activity after culture on glucose (Test $T_2$): at least 40 units, and preferably at least 80 units,

d) that the haploids thus selected are crossed with one another,

e) that, among the hybrids obtained from these crossings, are selected the strains according to claim 1 using the following tests:

- development, in the blue coloration test carried out on a Petri dish, of a blue colored culture after spreading on a YEG Petri dish in the presence of X alpha-glu,
- lack of development of a pink color in the test for determining invertase level according to Trumbly,
- maltose-permease activity after culture of the yeast on glucose medium, in the absence of maltose (Test $T_1$), of at least 9 units,
- maltose-permease activity after culture on maltose medium (Test $T'_1$), of at least 12 units,
- maltase activity on glucose medium, in the absence of maltose (Test $T_2$), of at least 50 units, preferably of at least 80 units and, still preferably, of at least 90 units,
- maltase activity on maltose medium (Test $T'_2$), of at least 200 units,
- invertase activity (Test $T_3$): less than 10 units, preferably more than 2 units,

and that an additional selection among the hybrids thus selected is carried out by conventional methods, i.e. by culture tests:

- as a function of the yield,
- as a function of nitrogen assimilation,
- as a function of the activities in Tests $A_1$ and $A_6$,
- as a function of the resistance to drying.

4. Process for the construction of new strains according to claim 3, characterized by the fact that the haploids (segregates) obtained from strains which have particularly remarkable properties on normal doughs and little osmosensitivity have their level of invertase activity lowered by the means of the technics of molecular biology.

5. New fresh bread-making yeast obtainable starting from the new strains of bread-making yeasts according to claims 1 or 2, characterized by the fact that

- in Test $A_1$ over 2 hours, it produces a release of gas comprised between 170 and 230 ml, preferably between 190 and 230 ml,
- in Test $A_5$ over 2 hours, it produces a release of gas comprised between 130 and 180 ml, preferably between 140 and 180 ml and, still more preferably, between 150 and 180 ml, and
- in a Test $A_6$ over 2 hours, it produces a release of gas comprised between 170 and 230 ml, preferably between 180 and 250 ml and, still more preferably, between 200 and 250 ml.

6. New dry bread-making yeast obtainable starting from the new strains of bread-making yeasts according to claims 1 or 2, characterized by the fact that

- in Test $A'_1$ over 2 hours, it produces a release of gas comprised between 120 and 145 ml, preferably between 130 and 145 ml and, still more preferably, between 135 and 145 ml,
- in Test $A'_5$ over 2 hours, it produces a release of gas comprised between 95 and 130 ml, preferably between 100 and 130 ml and, still more preferably, between 110 and 130 ml, and
- in a Test $A'_6$ over 2 hours, it produces a release of gas comprised between 135 and 190 ml, preferably between 140 and 190 ml and, still more preferably, between 150 and 190 ml.

7. Haploid deposited in the Collection Nationale de Cultures de Microorganismes (CNCM) at the Pasteur Institute under the No. I-1076.

8. Haploid deposited in the Collection Nationale de Cultures de Microorganismes (CNCM) at the Pasteur Institute under the No. I-1075.

9. Haploid deposited in the Collection Nationale de Cultures de Microorganismes (CNCM) at the Pasteur Institute under the No. I-1074.

**10.** Process according to one of claims 3 and 4, characterized by the fact that, if the haploids obtained from strains which are capable of giving fresh yeasts having a low osmosensitivity and producing at least 170 ml and preferably at least 180 ml in 2 hours in Test $A_1$:

- on the one hand, in the blue coloration test carried out on a Petri dish, are coloured blue in the test with coloring substance X alpha-glu and have a maltose-permease activity after culture on glucose (Test $T_1$) of at least 7 units and a maltase activity after culture on glucose (Test $T_2$) of at least 80 units,
- on the other hand, have an invertase level (Test $T_3$) more than 10 units,

the said invertase activity is lowered by disruption of an SUG gene of the said haploids.

**11.** Process according to one of claims 3, 4 and 10, characterized by the fact that the haploids which in addition have the following enzymatic activities are selected:

- maltose-permease activity after culture on maltose (Test $T'_1$): at least 12 units,
- maltase activity after culture on maltose (Test $T'_2$): at least 200 units.

**12.** Process according to one of claims 4, 10 and 11, characterized by the fact that, among the hybrids obtained, those are selected

- which give rise, in the blue coloration test carried out on a Petri dish, to the development of a blue colored culture after they have been spread on a YEG Petri dish in the presence of X alpha-glu,
- which do not give rise to the development of a pink color in the test for determining the invertase level according to Trumbly, and
- which have the following enzymatic activities:

  . maltose-permease activity after culture on glucose medium, in the absence of maltose (Test $T_1$) of at least 9 units,
  . maltose-permease activity after culture on maltose medium (Test $T'_1$) of at least 12 units,
  . maltase activity on glucose medium, in the absence of maltose (Test $T_2$) of at least 50 units, preferably of at least 80 units and, still preferably, of at least 90 units,
  . maltase activity on maltose medium (Test $T'_2$) of at least 200 units,
  . invertase activity (Test $T_3$) of less than 10 units, preferably of more than 2 units,

it being understood that, among the hybrids thus selected, an additional selection is carried out by conventional methods:

- as a function of the yield,
- as a function of nitrogen assimilation,
- as a function of the activities in Tests $A_1$ and $A_6$,
- as a function of the resistance to drying.

**13.** Process for the construction of new broad spectrum strains of bread-making yeasts according to claim 12, characterized by the fact that, if a hybrid obtained satisfies all the selection tests according to claim 12, with the exception of that concerning the invertase activity, this invertase activity is lowered by disruption of at least one of the SUC genes of said hybrid.

**14.** Process for the construction of new broad spectrum strains of bread-making yeasts having

- a high multiplication yield,
- a good nitrogen assimilation,
- a good glucose fermentation activity
- preferably a good resistance to drying,

and having simultaneously the following enzymatic activities:

- maltose-permease activity after growth of the yeast on glucose medium, in the absence of maltose (Test $T_1$): at least 9 units,

- maltase activity after growth of the yeast on glucose medium, in the absence of maltose (Test $T_2$): at least 80 units and, preferably, at least 90 units,
- invertase activity (Test $T_3$): less than 10 units and, preferably, more than 2 units,

starting from a strain having

- a high multiplication yield,
- a good nitrogen assimilation,
- a good glucose fermentation activity
- preferably a good resistance to drying,

and having simultaneously the following enzymatic activities:

- maltose-permease activity after growth of the yeast on glucose medium, in the absence of maltose (Test $T_1$): at least 9 units,
- maltase activity after growth of the yeast on glucose medium, in the absence of maltose (Test $T_2$): at least 80 units and, preferably, at least 90 units,
- invertase activity (Test $T_3$): more than 10 units,

characterized by the fact that its invertase activity is lowered to a level less than 10 units and preferably more than 2 units, by disruption of one or several genes coding for invertase (SUC gene).

15. Process for the construction of new broad spectrum strains of bread-making yeasts according to one of claims 1 and 2, characterized by the fact that at least two of the haploids according to claims 7 to 9 are crossed with one another and that, among the hybrids obtained from these crossings, there are selected those

- which give rise to the development, in the blue coloration test carried out on a Petri dish, of a blue colored culture after they have been spread on a YEG Petri dish in the presence of X alpha-glu,
- which do not give rise to the development of a pink color in the test for determining the invertase level according to Trumbly, and
- which have the following enzymatic activities:

   - maltose-permease activity after culture on glucose medium, in the absence of maltose (Test $T_1$): at least 9 units,
   - maltose-permease activity after culture on maltose medium (Test $T'_1$): at least 12 units,
   - maltase activity on glucose medium, in the absence of maltose (Test $T_2$): at least 50 units, preferably at least 80 units and, still preferably, at least 90 units,
   - maltase activity on maltose medium (Test $T'_2$): at least 200 units,
   - invertase activity (Test $T_3$): less than 10 units, preferably more than 2 units,

it being understood that, among the hybrids thus selected, an additional selection is carried out by conventional methods:

- as a function of the yield,
- as a function of nitrogen assimilation,
- as a function of the activities in Tests $A_1$ and $A_6$,
- as a function of the resistance to drying.

16. New strain of bread-making yeast deposited in the Collection Nationale de Cultures de Microorganismes at the Pasteur Institute under the No.I-1073.

17. New strain of bread-making yeast deposited in the Collection Nationale de Cultures de Microorganismes at the Pasteur Institute under the No.I-1072.

18. New strain of bread-making yeast deposited in the Collection Nationale de Cultures de Microorganismes at the Pasteur Institute under the No.I-1071.

19. New strain of bread-making yeast deposited in the Collection Nationale de Cultures de Microorganismes at the Pasteur Institute under the No.I-1202 and at the National Collection of Yeast Cultures under the No. NCYC 2383.

**20.** New strain of bread-making yeast obtained by the process according to claim 14.